# EUROPEAN PATENT SPECIFICATION

(11) **EP 4 386 751 B1**
(45) Date of publication and mention of the grant of the patent: **18.02.2026**
(21) Application number: 23195992.5
(22) Date of filing: 07.09.2023
(51) Int. Cl.: G10L 25/66, A61B 5/00, G16H 50/20, G16H 40/63, G16H 50/70, G10L 25/30

(54) **PARKINSON'S DESEASE PREDICTION APPARATUS AND PARKINSON'S DISEASE PREDICTION METHOD**
VORRICHTUNG ZUR VORHERSAGE DES PARKINSON-DESEASE UND VERFAHREN ZUR VORHERSAGE DER PARKINSON-KRANKHEIT
APPAREIL DE PRÉDICTION DE LA MALADIE DE PARKINSON ET PROCÉDÉ DE PRÉDICTION DE LA MALADIE DE PARKINSON

(30) Priority: 16.12.2022 KR 20220177620; 05.04.2023 KR 20230044734
(43) Date of publication of application: 19.06.2024
(73) Proprietor: Emocog Co., Ltd., Seoul 08813 (KR)
(72) Inventor: NOH, Yoo Hun, 06346 Seoul (KR); JO, Joon Sang, 03741 Seoul (KR); KIM, Hai Rin, 08830 Seoul (KR)
(74) Representative: Meissner Bolte Partnerschaft mbB

(56) References cited:
- CN-A- 114 373 484
- FAGHERAZZI GUY ET AL: "Voice for Health: The Use of Vocal Biomarkers from Research to Clinical Practice", vol. 5, no. 1, 16 April 2021 (2021-04-16), pages 78 - 88, XP055851902, Retrieved from the Internet <URL:https://www.karger.com/Article/Pdf/515346> [retrieved on 20211015], DOI: 10.1159/000515346
- VARALAKSHMI P ET AL: "Parkinson Disease Detection Based On Speech Using Various Machine Learning Models and Deep Learning Models", 2021 INTERNATIONAL CONFERENCE ON SYSTEM, COMPUTATION, AUTOMATION AND NETWORKING (ICSCAN), IEEE, 30 July 2021 (2021-07-30), pages 1 - 6, XP033969277, DOI: 10.1109/ICSCAN53069.2021.9526372
- HAO FANG ET AL: "Parkinsonian Chinese Speech Analysis towards Automatic Classification of Parkinson's Disease", ARXIV.ORG, CORNELL UNIVERSITY LIBRARY, 201 OLIN LIBRARY CORNELL UNIVERSITY ITHACA, NY 14853, 31 May 2021 (2021-05-31), XP081972374

## Description

### BACKGROUND

### 1. Field

The disclosure relates to a Parkinson's disease prediction apparatus and a Parkinson's disease prediction method.

### 2. Description of the Related Art

Parkinson's disease is one of the degenerative brain diseases and may refer to a disease that causes a movement disorder due to destruction of dopaminergic nerves of the substantia nigra present in the center of the brainstem.

It is known that most patients with Parkinson's disease suffer from speech problems. This may mean that the presence or absence of Parkinson's disease may be indirectly diagnosed from speech methods of patients who may have Parkinson's disease or sentences uttered by the patients.

The background art is technical information possessed by the inventors for the derivation of the disclosure or obtained during the derivation of the disclosure, and is not necessarily known technology disclosed to the general public prior to the filing of the disclosure.

The disclosure pertains to a technology developed through Project Number SU220002, "Bizcall-Based Senior Citizen Optimization Technology Commercialization of Dementia Early Screening Technology" of the 2022 growth stage scale-up technology commercialization support project of the Seoul Business Agency, Seoul.

CN 114 373 484 A relates to a medical application in the field of deep learning and relates to small sample learning method and system for speech driven Parkinson's disease multi-symptom characteristic parameters.

FAGHERAZZI GUY ET AL: "Voice for Health: The Use of Vocal Biomarkers from Research to Clinical Practice", DIGITAL BIOMARKERS, vol. 5, no. 1, 16 April 2021 (2021-04-16), pages 78-88, DOI: 10.1159/000515346, URL:https://www.karger.com/Article/Pdf/515346 discloses various applications of voice for health-related purposes.

VARALAKSHMI P ET AL: "Parkinson Disease Detection Based On Speech Using Various Machine Learning Models and Deep Learning Models", 2021 INTERNATIONAL CONFERENCE ON SYSTEM, COMPUTATION, AUTOMATION AND NETWORKING (ICSCAN), IEEE, 30 July 2021 (2021-07-30), pages 1-6, DOI: 10.1109/ICSCAN53069.2021.9526372 describes a model which can predict Parkinson's disease with high performance metrics.

HAO FANG ET AL: "Parkinsonian Chinese Speech Analysis towards Automatic Classification of Parkinson's Disease", ARXIV.ORG, CORNELL UNIVERSITY LIBRARY, 201 OLIN LIBRARY CORNELL UNIVERSITY ITHACA, NY 14853, 31 May 2021 (2021-05-31) discloses a classical machine learning method with ranking algorithm for feature selection, convolutional and recurrent deep networks.

### SUMMARY

The disclosure aims to provide a Parkinson's disease prediction apparatus and a Parkinson's disease prediction method that predict Parkinson's disease of a speaker through analysis of a speaker's audio.

Objectives of the disclosure are not limited to those described above, and other objectives and advantages of the disclosure not described herein will be understood from the following description and will be more clearly understood from embodiments. In addition, it will be apparent that the objectives and advantages to be achieved by the disclosure may be realized by means presented in the claims and combinations thereof.

Additional aspects will be set forth in part in the description which follows and, in part, will be apparent from the description, or may be learned by practice of the presented embodiments of the disclosure.

A Parkinson's disease prediction method according to the present embodiment is performed by a processor of a Parkinson's disease prediction apparatus and includes extracting syntactic combinations from audio data including a speaker's speech result, verifying accuracy of a Parkinson's disease prediction model by changing conditions for preprocessing the audio data and the syntactic combinations, determining a syntactic combination ranked in a high rank as audio data for Parkinson's disease prediction, based on a result of verifying the accuracy of the Parkinson's disease prediction model, and inputting, to the Parkinson's disease prediction model, a speaker's speech result corresponding to the audio data for the Parkinson's disease prediction and obtaining a Parkinson's disease prediction result for the speaker as an output of the Parkinson's disease prediction model.

A Parkinson's disease prediction apparatus includes a processor and a memory operatively connected to the processor and configured to store at least one code executable by the processor, wherein, when executed by the process, the at least one code causes the processor to extract syntactic combinations from audio data including a speaker's speech result, verify accuracy of a Parkinson's disease prediction model by changing conditions for preprocessing the audio data and the syntactic combinations, determine a syntactic combination ranked in a high rank as audio data for Parkinson's disease prediction, based on a result of verifying the accuracy of the Parkinson's disease prediction model, and inputting, to the Parkinson's disease prediction model, a speaker's speech result corresponding to the audio data for the Parkinson's disease prediction and obtain a Parkinson's disease prediction result for the speaker as an output of the Parkinson's disease prediction model.

In addition, the disclosure may further provide another method of implementing the disclosure, another system for implementing the disclosure, and a computer-readable recording medium having recorded thereon a computer program for executing the method.

Other aspects, features, and advantages of the disclosure will become better understood through the accompanying drawings, the appended claims, and the detailed description.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a schematic block diagram for describing a configuration of a Parkinson's disease prediction apparatus according to the present embodiment;
FIG. 2 is a diagram illustrating an example of a language regulation showing the number and order of Korean consonants and vowels, according to the present embodiment;
FIGS. 3 to 5 are diagrams illustrating an example of syntactic combinations extracted by the Parkinson's disease prediction apparatus according to the present embodiment;
FIGS. 6 to 32 are diagrams for describing accuracy verification of the Parkinson's disease prediction apparatus according to the present embodiment;
FIG. 33 is a schematic block diagram for describing a configuration of a Parkinson's disease prediction apparatus according to another embodiment; and
FIG. 34 is a flowchart for describing a Parkinson's disease prediction method according to the present embodiment.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Advantages and features of the disclosure, and methods of achieving them will be clarified with reference to embodiments described below in detail with reference to the accompanying drawings. However, it should be understood that the disclosure is not limited to the embodiments presented below, but may be implemented in various different forms and may include any modifications, equivalents, and substitutes included in the scope of the disclosure. Embodiments presented below are provided so that the disclosure will be thorough and complete and will fully convey the concept of the embodiments to those of ordinary skill in the art. In describing the disclosure, when the detailed description of the relevant known technology is determined to obscure the gist of the disclosure, the detailed description thereof may be omitted.

The terms as used in the present specification are only used to describe specific embodiments and are not intended to limit the disclosure. The singular forms as used herein are intended to include the plural forms as well unless the context clearly indicates otherwise. The terms "comprise," "include," or "have" as used in the present application are inclusive and therefore specify the presence of one or more stated features, integers, steps, operations, elements, components, or any combination thereof, but do not preclude the presence or addition of one or more other features, integers, steps, operations, elements, components, or any combination thereof. While the terms "first," "second," etc. may be used herein to describe various elements, such elements should not be limited by these terms. These terms are only used to distinguish one element from another.

In addition, the term "unit" as used herein may be a hardware component such as a processor or a circuit, and/or a software component to be executed by the hardware component such as a processor.

In addition, in the present application, brackets ([ ]) may include English representations of syllables, syntactics, or syntactic combinations before the brackets. And the English notations may represent the Korean pronunciation of the syllables, the phrases, or the combinations of phrases. The syllable may refer to a minimum unit of speech that is most easily recognized intuitively during speech. In the present embodiment, the syllable may include, for example, "[ga]," "[ha]," and the like. In addition, the syntactic may be a spacing unit as a minimum unit of a sentence component. In the present embodiment, the syntactic may include, for example, "[aeiou]," "[nanana]," "[rarara]," and the like. In addition, the syntactic combination may include a result of combining one or more syntactics. In the present embodiment, the syntactic combination may include, for example, "[gagagananana]," "[aeiougagagananana]," "[nananasasasa]," and the like.

Hereinafter, the embodiments will be described in detail with reference to the accompanying drawings. When describing the embodiments with reference to the accompanying drawings, the same or corresponding elements are denoted by the same reference numerals and redundant descriptions thereof are omitted.

In the following embodiments, the terms "first," "second," etc. are not used in a restrictive sense and are used to distinguish one element from another.

In the following embodiments, the singular forms are intended to include the plural forms as well unless the context clearly indicates otherwise.

In the following embodiments, the terms "comprise," "include," or "have" specify the presence of stated features or elements, but do not preclude the presence or addition of one or more other features or elements.

When a certain embodiment may be implemented differently, a specific process order may be performed differently from the described order. For example, two consecutively described processes may be performed substantially at the same time or performed in an order opposite to the described order.

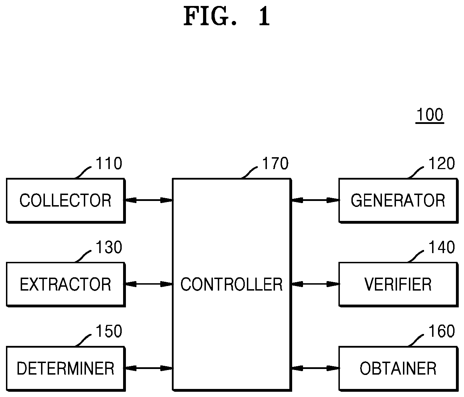

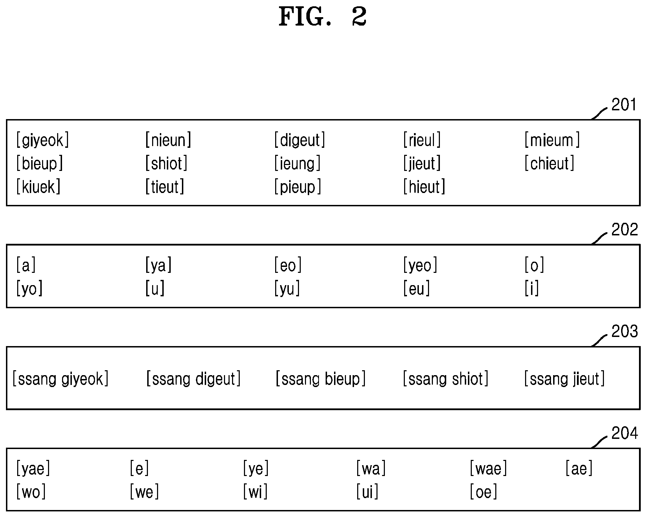

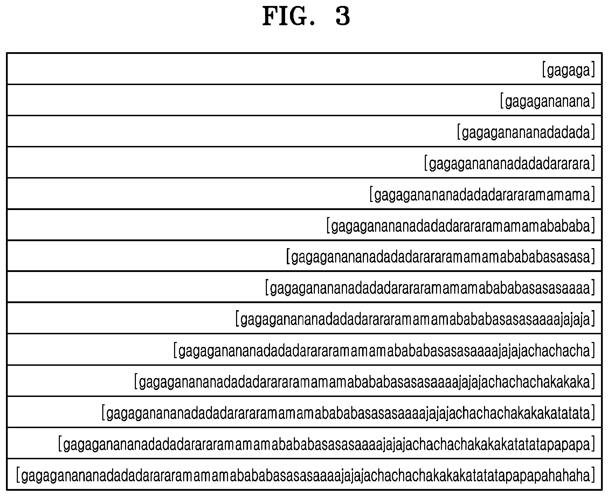

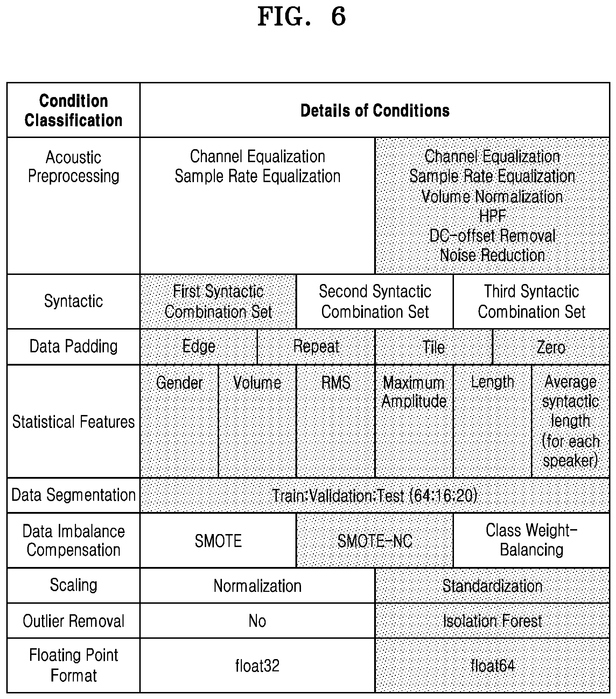

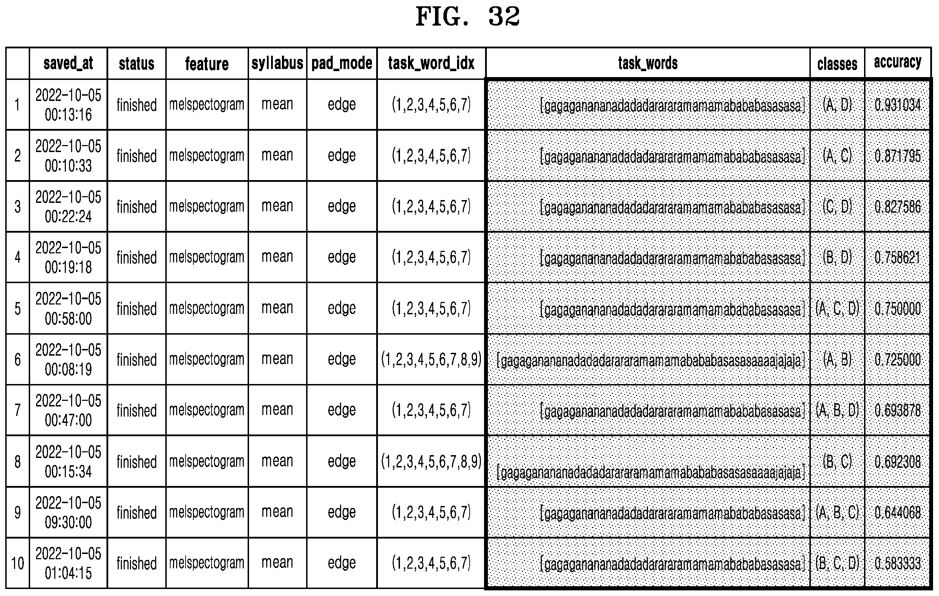

FIG. 1 is a schematic block diagram for describing a configuration of a Parkinson's disease prediction apparatus 100 according to the present embodiment, FIG. 2 is a diagram illustrating an example of a language regulation showing the number and order of Korean consonants and vowels, according to the present embodiment, FIGS. 3 to 5 are diagrams illustrating an example of syntactic combinations extracted by the Parkinson's disease prediction apparatus 100 according to the present embodiment, and FIGS. 6 and 32 are diagrams for describing accuracy verification of the Parkinson's disease prediction apparatus 100 according to the present embodiment.

The Parkinson's disease prediction apparatus 100 according to the present embodiment may be present independently in the form of a server, or a Parkinson's disease prediction function provided by the Parkinson's disease prediction apparatus 100 may be implemented in the form of an application and mounted on a user terminal (not shown). The user terminal may receive a Parkinson's disease prediction service by accessing a Parkinson's disease prediction site and/or a Parkinson's disease prediction application provided by the Parkinson's disease prediction apparatus 100.

Referring to FIGS. 1 to 32, the Parkinson's disease prediction apparatus 100 may include a collector 110, a generator 120, an extractor 130, a verifier 140, a determiner 150, an obtainer 160, and a controller 170.

The collector 110 may collect audio data including a speaker's speech result. In the present embodiment, the audio data may include a voice recording file in which the reading of preset materials is recorded. The preset materials may include documents, files, etc. including syllables, syntactics, or syntactic combinations to be described below. In addition, the audio data may include data obtained by uttering one of syllables, syntactics, or syntactic combinations to be described below for a preset time (e.g., 1-2 seconds). Moreover, the audio data may include data obtained by uttering syllables, syntactics, or syntactic combinations to be described below in a high pitch and data obtained by uttering syllables, syntactics, or syntactic combinations to be described below in a low pitch.

In the present embodiment, audio data and audio signals may be used interchangeably with the same meaning.

The generator 120 may generate a Parkinson's disease prediction model. In the present embodiment, the Parkinson's disease prediction model may be generated by training a deep neural network model pre-trained to predict Parkinson's disease for the speaker by using the audio data including the speaker's speech result. The deep neural network model may be a model that receives the audio data including the speaker's speech result and is trained in a supervised learning method by using training data labeled with one of normal, Parkinson, multiple system atrophy, and cerebellar atrophy included in the Parkinson's disease-related diseases.

The generator 120 may train an initially configured deep neural network model in a supervised learning method by using the labeled training data. The initially configured deep neural network model is an initial model designed to be configured as a Parkinson's disease prediction model, and parameter values are in a state of being set to arbitrary initial values. As the initially configured deep neural network model is trained by using the training data, the parameter values may be optimized. Therefore, the initially configured deep neural network model may be completed as a Parkinson's disease prediction model capable of accurately predicting Parkinson's disease for the speaker.

The extractor 130 may extract syntactic combinations from the audio data including the speaker's speech result. In the present embodiment, the syntactic combinations may include a plurality of first different syntactic combinations, a plurality of second different syntactic combinations, and a plurality of third different syntactic combinations.

The first different syntactic combinations that are extracted from the audio data by the extractor 130 may include at least one first syllable obtained by combining a certain consonant with a certain vowel.

The certain consonant included in the first different syntactic combinations may be combined with the certain vowel in the consonant order according to the language regulation. FIG. 2 illustrates a consonant order 201 according to the language regulation. Referring to FIG. 2, the consonant order 201 may be "[giyeok], [nieun], [digeut], [rieul], [mieum], [bieup], [shiot], [ieung], [jieut], [chieut], [kiuek], [tieut], [pieup], and [hieut]."

The certain vowel included in the first different syntactic combinations may be determined as a single vowel. In the present embodiment, the certain vowel may be "[a]," which is the first vowel in a vowel order 202 disclosed in the language regulation illustrated in FIG. 2.

The extractor 130 may extract, from the audio data, a first syntactic combination in which a first syllable generated by combining n^{th} to (n+k)^{th} consonants according to the consonant order with the certain vowel is repeated a preset number of times (e.g., three times). Here, n may include a natural number and k may include 0 and a natural number.

In an embodiment, when n=1 and k=0, the first syntactic combination may be "[gagaga]." In the present embodiment, " 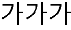 [gagaga]" may also be referred to as a syntactic according to the above definition. In another embodiment, when n=1 and k=3, the first syntactic combination may be "[gagaganananadadadararara]."

FIG. 3 illustrates the first syntactic combination that is extracted from the audio data by the extractor 130. Referring to FIG. 3, the first syntactic combination may include "[gagaga]" ... "[gagaga...hahaha]."

The second different syntactic combinations that are extracted from the audio data by the extractor 130 may be a combination of one or more of a basic vowel set, a second syllable, and a first syntactic combination.

In the present embodiment, the basic vowel set may include "[aeiou]." In the present embodiment, the second syllable may be generated by combining a certain double consonant with a certain vowel. In the present embodiment, the certain double consonant included in the second syllable may be "[ssang bieup]," which is the third double consonant in a double consonant order 203 according to the language regulation illustrated in FIG. 2. In addition, the certain vowel included in the second syllable may be "[wa]," which is the fourth double vowel in a double vowel order 204 according to the language regulation illustrated in FIG. 2. Consequently, the second syllable may be "[ppawa]." In the present embodiment, the second syllable may be repeated a preset number of times (e.g., three times) in order to be included in the second syntactic combination.

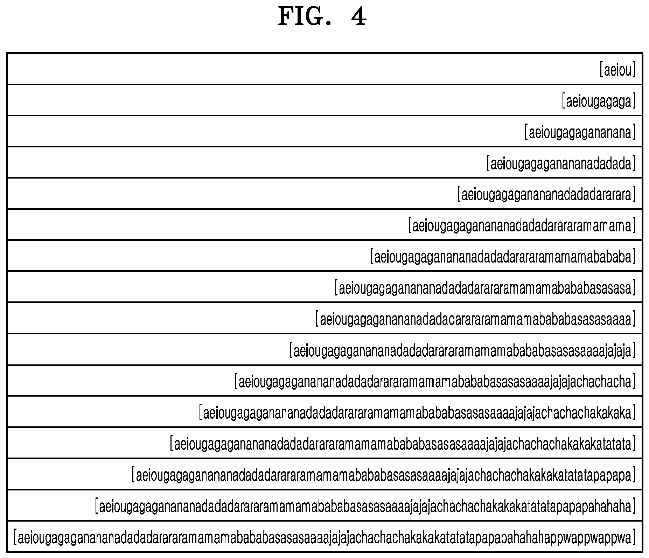

FIG. 4 illustrates the second syntactic combination that is extracted from the audio data by the extractor 130. Referring to FIG. 4, the second syntactic combination may include "[aeiou]" ... "[aeiou... ppawappawappawa]."

The third different syntactic combinations that are extracted from the audio data by the extractor 130 may include a third syllable and one syntactic combination included in the second different syntactic combinations.

In the present embodiment, the third syllable may be generated by combining a certain consonant with a certain vowel. In the present embodiment, the certain consonant included in the third syllable may be specified as "[nieun], [digeut], [shiot], [ieung], and [hieut]" in the consonant order 201 according to the language regulation illustrated in FIG. 2. In addition, the certain vowel included in the third syllable may be "[a]," which is the first vowel in the vowel order 202 according to the language regulation illustrated in FIG. 2. Consequently, the third syllable may be specified as "[na]," "[da]," "[sa]," "[a]," and "[ha]". In the present embodiment, the third syllable may be repeated a preset number of times (e.g., three times) in order to be included in the third syntactic combination.

In addition, in the present embodiment, the third syntactic combination may be specified as "[aeiougagaga...mamama]," which is one syntactic combination included in the second syntactic combinations.

In the present embodiment, unlike the first different syntactic combinations and the second different syntactic combinations, the third different syntactic combinations may be specified as syntactic combinations ranked in the top first to fourth rank in terms of the accuracy of the Parkinson's disease prediction model after augmenting the audio data.

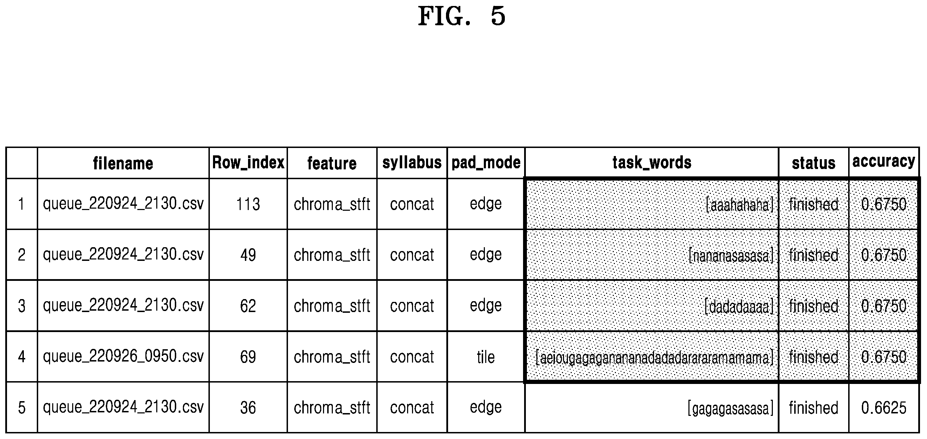

FIG. 5 illustrates the third different syntactic combinations that are extracted from the audio data by the extractor 130. Referring to FIG. 5, the third different syntactic combinations may be specified as "[nananasasasa]," "[dadadaaaa]," and "[aaahahaha]," which are combinations obtained by repeating the third syllable a preset number of times, and "[aeiougagaga... mamama]" included in the second different syntactic combinations.

The verifier 140 may verify the accuracy of the Parkinson's disease prediction model by changing conditions for preprocessing the audio data and the syntactic combinations.

In the present embodiment, the conditions for preprocessing the audio data may include a condition for executing one or more of acoustic preprocessing, syntactics, data padding, statistical features, data segmentation, label imbalance compensation, scaling, outlier removal, and floating point format.

The acoustic preprocessing may be classified into first acoustic preprocessing and second acoustic preprocessing. The controller 170 may select one of the first acoustic preprocessing and the second acoustic preprocessing and output the selected one of the first acoustic preprocessing and the second acoustic preprocessing to the verifier 140. The verifier 140 may verify the accuracy of the Parkinson's disease prediction model by executing the selected one of the first acoustic preprocessing and the second acoustic preprocessing.

In the present embodiment, the first acoustic preprocessing may include channel equalization processing and sample rate equalization processing. The channel equalization processing may mean that channels for recording audio data are unified into one (mono) channel. The sample rate equalization processing may mean that sample rates are unified into a certain number of times per second (e.g., 16,000 times). The sample rate may refer to the number of samplings per time when an analog audio signal is converted into a digital audio signal.

In the present embodiment, the second acoustic preprocessing may include channel equalization processing, sample rate equalization processing, volume normalization processing, high-pass filtering processing, DC-offset removal processing, and noise reduction (stationary, 40%) processing. Because the channel equalization processing and the sample rate equalization processing are the same as those described above, a detailed description thereof is omitted. The volume of the audio data may vary depending on a speaker's voice volume or how far away the speaker is from a recording device (not shown) during recording. The volume normalization processing may refer to normalizing entire audio data according to the average volume of the audio data in order to make the volume of differently recorded audio data relatively uniform. The high-pass filtering processing may refer to passing only an audio signal having a frequency higher than a cutoff frequency (e.g., 90 Hz). The DC-offset removal processing may refer to making a DC-offset zero. The DC-offset may represent the average intensity of waveforms. The DC-offset may not be zero. In this case, it may be said that the DC-offset has occurred. The DC-offset may occur when the voice is not properly recorded or when the recording device is in a poor state. The noise reduction processing may refer to reducing noise in order to obtain a clean signal. The stationary may refer to applying a noise threshold to all sections of the audio signal, and 40% may refer to reducing the amplitude of the audio signal to 40 % when noise is detected.

The syntactics may be classified into a plurality of first different syntactic combinations, a plurality of second different syntactic combinations, and a plurality of third different syntactic combinations. The controller 170 may select one or more of the first different syntactic combinations, the second different syntactic combinations, and the third different syntactic combinations and output the selected one or more of the first different syntactic combinations, the second different syntactic combinations, and the third different syntactic combinations to the verifier 140. The verifier 140 may verify the accuracy of the Parkinson's disease prediction model by executing the selected one or more of the first different syntactic combinations, the second different syntactic combinations, and the third different syntactic combinations. In the present embodiment, for convenience of explanation, the syntactics may be excluded from the conditions for processing the audio data.

The data padding may refer to equalizing the size of data by filling a blank section with a specific value before and after the data. In the present embodiment, the data padding may include audio data padding. The audio data padding may refer to equalizing the size of audio data by filling a silent section with a specific value before and after the audio data. Types of data padding may include edge, repeat, tile, and zero. The edge may fill a previous silent section with a start value of the data and may fill a subsequent silent section with an end value of the data. The repeat may fill a silent section with a repeated individual value (e.g., 1). The tile may fill a silent section with a repeated whole value (e.g., 1234). The zero may fill a silent section with zero. The controller 170 may select one or more of the types of data padding and output the selected one or more of the types of data padding to the verifier 140. The verifier 140 may verify the accuracy of the Parkinson's disease prediction model by executing the selected one or more of the types of data padding.

The statistical features may include at least one of a gender, a volume, a magnitude (root mean square), a maximum amplitude, a length, and an average syntactic length for each speaker, which may be extracted from the audio data. The controller 170 may select one or more of the statistical features and output the selected one or more of the statistical features to the verifier 140. The verifier 140 may verify the accuracy of the Parkinson's disease prediction model by executing the selected one or more of the statistical features.

The data segmentation may include segmenting the entire data set used in the Parkinson's disease prediction model in the form of a certain ratio. In the present embodiment, the entire data set may be segmented into a train set, a validation set, and a test set. For example, the train set, the validation set, and the test set may be segmented into 64:16:20. The controller 170 may determine a segmentation ratio of the entire data set and output the determined segmentation ratio to the verifier 140. The verifier 140 may verify the accuracy of the Parkinson's disease prediction model by segmenting the audio data at the determined segmentation ratio (64:16:20) of the entire data set.

The data imbalance compensation may refer to compensating imbalance by augmenting the audio data. In order to compensate for data imbalance, one or more of a synthetic minority over sampling technique (SMOTE) algorithm, a synthetic minority over sampling technique of nominal and continuous (SMOTE-NC) algorithm, and a class weight-balancing algorithm may be used. The SMOTE algorithm may newly generate and augment data of a class existing at a low ratio by using a k-NN algorithm. The SMOTE-NC algorithm may be used for data in which categorical data and continuous data are mixed. The class weight-balancing algorithm may adjust the training of the Parkinson's disease prediction model by making data with a lower class ratio have a greater loss weight. The controller 170 may select one or more of the SMOTE algorithm, the SMOTE-NC algorithm, and the class weight-balancing algorithm and output the selected one or more of the SMOTE algorithm, the SMOTE-NC algorithm, and the class weight-balancing algorithm to the verifier 140. The verifier 140 may verify the accuracy of the Parkinson's disease prediction model by executing the selected one or more of the SMOTE algorithm, the SMOTE-NC algorithm, and the class weight-balancing algorithm.

The scaling may refer to adjusting a range of a data value. The reasons for scaling the data are that, when the data value is too large or too small, the data value may converge to 0 or diverge to infinity during the model training process. In the present embodiment, the scaling may be classified into normalization or standardization scaling. The normalization may refer to executing scaling so that a feature value exists between 0 and 1. The standardization may refer to executing scaling so that an average of feature values becomes 0 and a variance of feature values becomes 1. The controller 170 may select normalization or standardization from among data scalings and output the selected normalization or standardization to the verifier 140. The verifier 140 may verify the accuracy of the Parkinson's disease prediction model by using the normalization or the standardization selected from among the data scalings.

The outlier removal may be executed in order to improve the accuracy of the model at the time of training. In the present embodiment, an isolation forest algorithm may be used as an outlier removal algorithm. The controller 170 may select whether or not to remove the outlier and output a selection result to the verifier 140. The verifier 140 may verify the accuracy of the Parkinson's disease prediction model by executing the selected outlier removal or non-removal.

The floating point format is a data representation method. In the present embodiment, the floating point format may be classified into float32 and float64. The float32 may represent data in 32 bits, and the float64 may represent data in 64 bits. The float32 may represent data with half the capacity of the float64, but may cause data loss. The float64 may represent data with less loss and in more detail. The controller 170 may select whether to use the float32 or the float64 for data representation and output a selection result to the verifier 140. The verifier 140 may verify the accuracy of the Parkinson's disease prediction model by representing the audio data with the selected float32 or float64.

In an alternative embodiment, the conditions for preprocessing the audio data may further include extracting audio feature values. In the present embodiment, methods of extracting the audio feature values may be classified into a short-time Fourier transform chromagram (STFT), a constant-Q transform chromagram (CQT), chroma energy normalized statistics (CENS), a mel-scaled spectrogram, mel-frequency cepstral coefficients (MFCC), and a tempogram. The STFT may divide an audio signal into sections with a certain length and extract a spectrum according to time by applying a Fourier transform to each section. The CQT may convert an audio signal into a frequency domain and extract a vector value representing a frequency distribution of a pitch. The CENS may extract a normalized chroma vector value by using short-term harmonics of an audio signal. The mel-scaled spectrogram may extract features by analyzing a mel-scaled spectrogram. The MFCC may extract a vector value of a feature sound by sampling audio data in units of certain time and then analyzing a spectrum. The tempogram may extract mid-level features representing local tempo characteristics of an audio signal. In the present embodiment, the controller 170 may set one or more of the methods of extracting the audio feature values to be performed by default and output the set one or more methods to the verifier 140. When verifying the accuracy of the Parkinson's disease prediction model, the verifier 140 may execute one or more of the methods of extracting the audio feature values by default. In an alternative embodiment, the controller 170 may select one or more of the methods of extracting the audio feature values and output the selected one or more methods to the verifier 140. The verifier 140 may verify the accuracy of the Parkinson's disease prediction model by executing the selected one or more of the methods of extracting the audio feature values.

In an alternative embodiment, the conditions for preprocessing the audio data may further include syllable processing. In the present embodiment, the syllable processing may be classified into mean processing and concatenation (concat) processing. The mean processing may include a process of obtaining an average of syllables uttered in a high pitch and syllables uttered in a low pitch. The concat processing may include a process of concatenating a syllable uttered in a low tone after a syllable uttered in a high tone. For example, in the case of concatenating "[gagaganana]" that is one of the first different syntactic combinations, it may be a result of concatenating a high-pitched "[gagaga]" with a low-pitched "[nanana]." In the present embodiment, the controller 170 may set one or more of the mean processing and the concat processing to be executed by default and output the set one or more of the mean processing and the concat processing to the verifier 140. When verifying the accuracy of the Parkinson's disease prediction model, the verifier 140 may execute one or more of the mean processing and the concat processing by default. In an alternative embodiment, the controller 170 may select one or more of the mean processing and the concat processing and output the selected one or more of the mean processing and the concat processing to the verifier 140. The verifier 140 may verify the accuracy of the Parkinson's disease prediction model by executing the selected one or more of the mean processing and the concat processing.

In the present embodiment, the verifier 140 may execute first to seventh verifications in order to verify the accuracy of the Parkinson's disease prediction model.

The verifier 140 may execute the first verification to verify the accuracy of the Parkinson's disease prediction model based on the first preprocessing condition for preprocessing the audio data and the first different syntactic combinations.

FIG. 6 is a table showing the first preprocessing condition for preprocessing the audio data in order to execute the first verification. Referring to FIG. 6, the first preprocessing condition may include a condition for executing second acoustic preprocessing from among the conditions for preprocessing the audio data, executing data padding including edge, repeat, tile, and zero, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64.

The verifier 140 may execute the second verification to verify the accuracy of the Parkinson's disease prediction model based on the second preprocessing condition for preprocessing the audio data and the second different syntactic combinations.

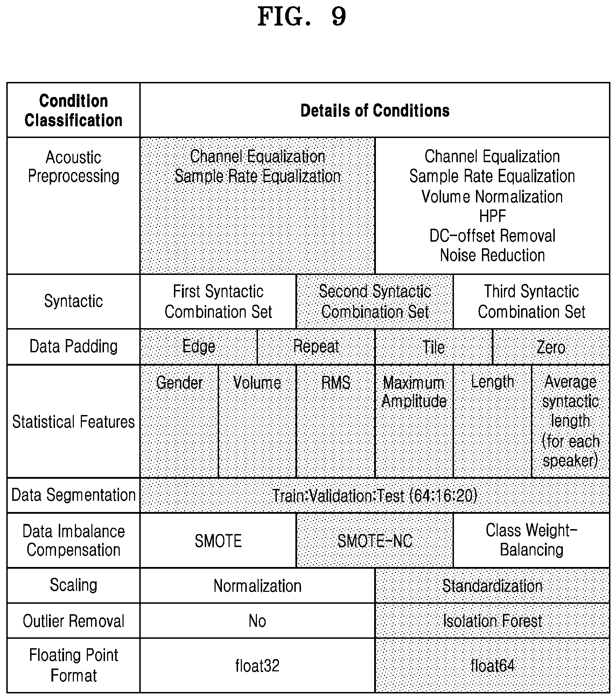

FIG. 9 is a table showing the second preprocessing condition for preprocessing the audio data in order to execute the second verification. Referring to FIG. 9, the second preprocessing condition may include a condition for executing first acoustic preprocessing from among the conditions for preprocessing the audio data, executing data padding including edge, repeat, tile, and zero, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64. In the present embodiment, the second verification may differ from the first verification in terms of the acoustic preprocessing and the syntactic combinations.

The verifier 140 may execute the third verification to verify the accuracy of the Parkinson's disease prediction model based on the third preprocessing condition for preprocessing the audio data and the third different syntactic combinations.

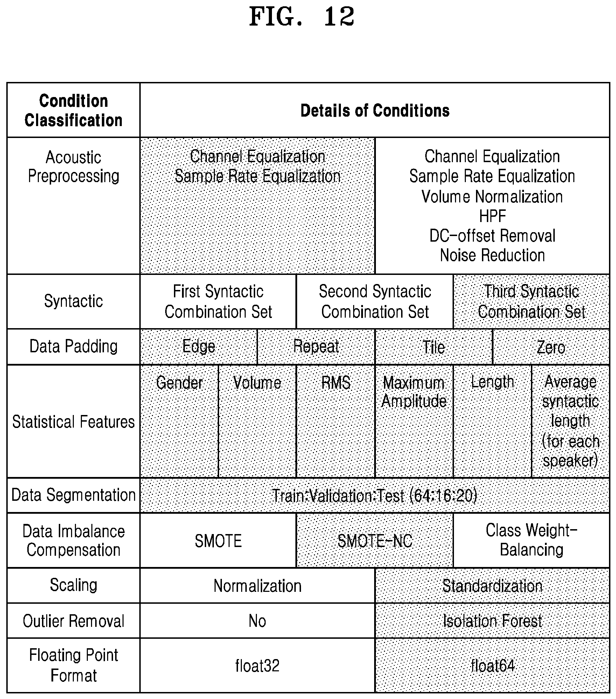

FIG. 12 is a table showing the third preprocessing condition for preprocessing the audio data in order to execute the third verification. Referring to FIG. 12, the third preprocessing condition may include a condition for executing first acoustic preprocessing from among the conditions for preprocessing the audio data, executing data padding including edge, repeat, tile, and zero, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64. In the present embodiment, the third verification may differ from the second verification in terms of the syntactic combinations.

The verifier 140 may execute the fourth verification to verify the accuracy of the Parkinson's disease prediction model based on the fourth preprocessing condition for preprocessing the audio data and the third different syntactic combinations.

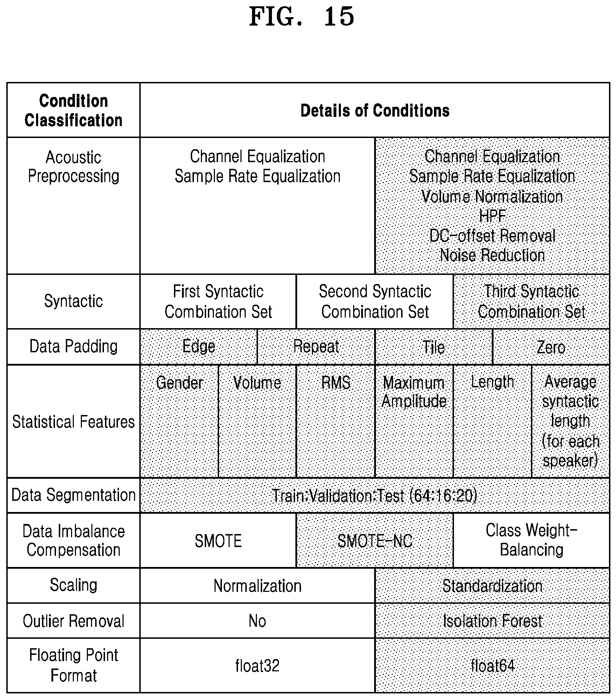

FIG. 15 is a table showing the fourth preprocessing condition for preprocessing the audio data in order to execute the fourth verification. Referring to FIG. 15, the fourth preprocessing condition may include a condition for executing second acoustic preprocessing from among the conditions for preprocessing the audio data, executing data padding including edge, repeat, tile, and zero, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64. In the present embodiment, the fourth verification may differ from the third verification in terms of the acoustic preprocessing.

The verifier 140 may execute the fifth verification to verify the accuracy of the Parkinson's disease prediction model based on the fifth preprocessing condition for preprocessing the audio data and the third different syntactic combinations.

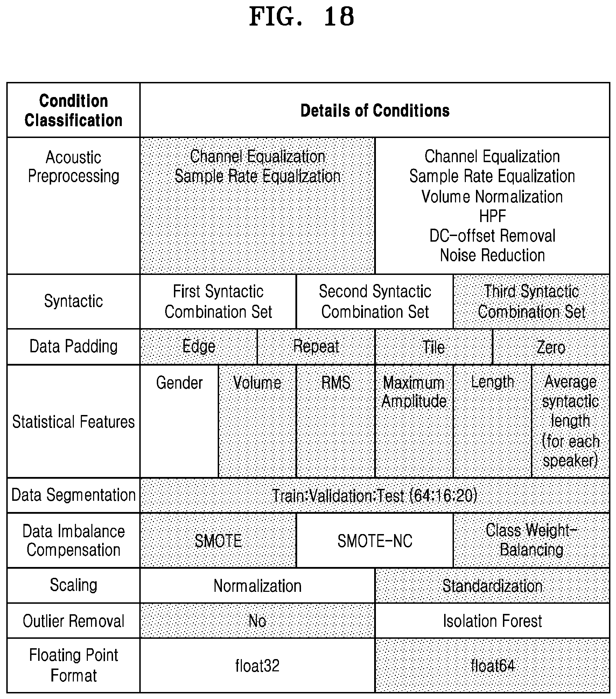

FIG. 18 is a table showing the fifth preprocessing condition for preprocessing the audio data in order to execute the fifth verification. Referring to FIG. 18, the fifth preprocessing condition may include a condition for executing first acoustic preprocessing from among the conditions for preprocessing the audio data, executing data padding including edge, repeat, tile, and zero, executing statistical features including a volume, a magnitude, a maximum amplitude, a length, and an average syntactic length for each speaker, executing data segmentation to segment a train set, a validation set, and a test set into 64:16:20, executing data imbalance compensation including an SMOTE algorithm and a class weight-balancing algorithm, executing scaling including standardization, executing outlier non-removal, and executing a floating point format including float64. In the present embodiment, the fifth verification may differ from the fourth verification in terms of the acoustic preprocessing, the statistical features, the data imbalance compensation, and the outlier removal.

The verifier 140 may execute the sixth verification to verify the accuracy of the Parkinson's disease prediction model based on the sixth preprocessing condition for preprocessing the audio data, the first different syntactic combinations, and the second different syntactic combinations.

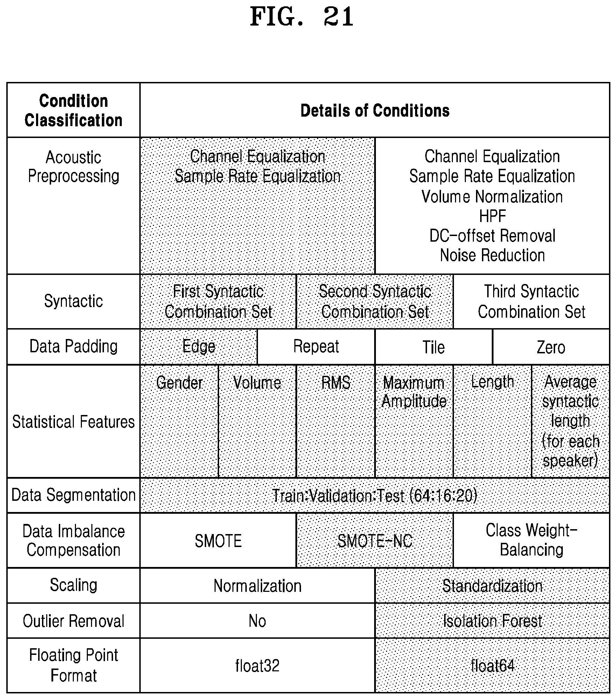

FIG. 21 is a table showing the sixth preprocessing condition for preprocessing the audio data in order to execute the sixth verification. Referring to FIG. 21, the sixth preprocessing condition may include a condition for executing first acoustic preprocessing from among the conditions for preprocessing the audio data, executing data padding including edge, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64. In the present embodiment, the sixth verification may differ from the fifth verification in terms of the syntactic combinations, the data padding, the statistical features, the data imbalance compensation, and the outlier removal.

The verifier 140 may execute the seventh verification to verify the accuracy of the Parkinson's disease prediction model based on the seventh preprocessing condition for preprocessing the audio data, the first different syntactic combinations, and the second different syntactic combinations.

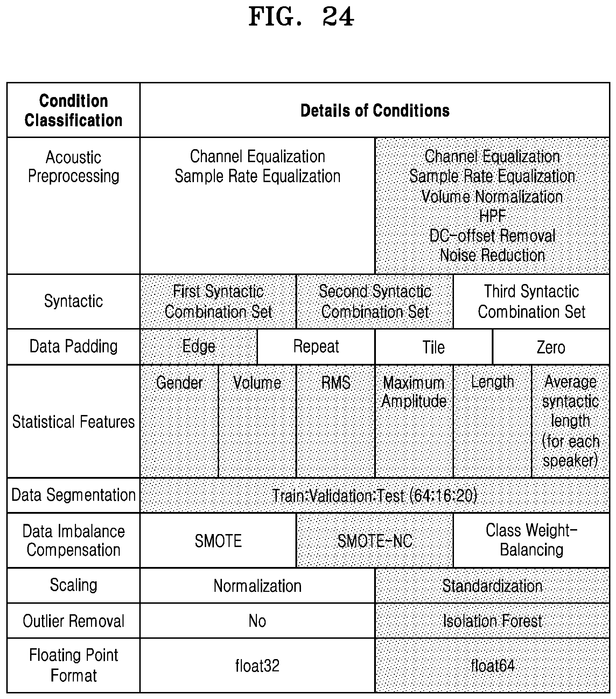

FIG. 24 is a table showing the seventh preprocessing condition for preprocessing the audio data in order to execute the seventh verification. Referring to FIG. 24, the seventh preprocessing condition may include a condition for executing second acoustic preprocessing from among the conditions for preprocessing the audio data, executing data padding including edge, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64. In the present embodiment, the seventh verification may differ from the sixth verification in terms of the acoustic preprocessing.

The determiner 150 may determine a syntactic combination ranked in a high rank as the audio data for the Parkinson's disease prediction, based on the result of verifying the accuracy of the Parkinson's disease prediction model, which is output from the verifier 140.

The determiner 150 may determine the first syntactic combination ranked in the highest rank as the audio data for the Parkinson's disease prediction, based on the result of the first verification output from the verifier 140. The first syntactic combination ranked in the highest rank may be a syntactic combination in which the first syllable generated by combining the first to eleventh consonants according to the consonant order with the certain vowel is repeated a preset number of times.

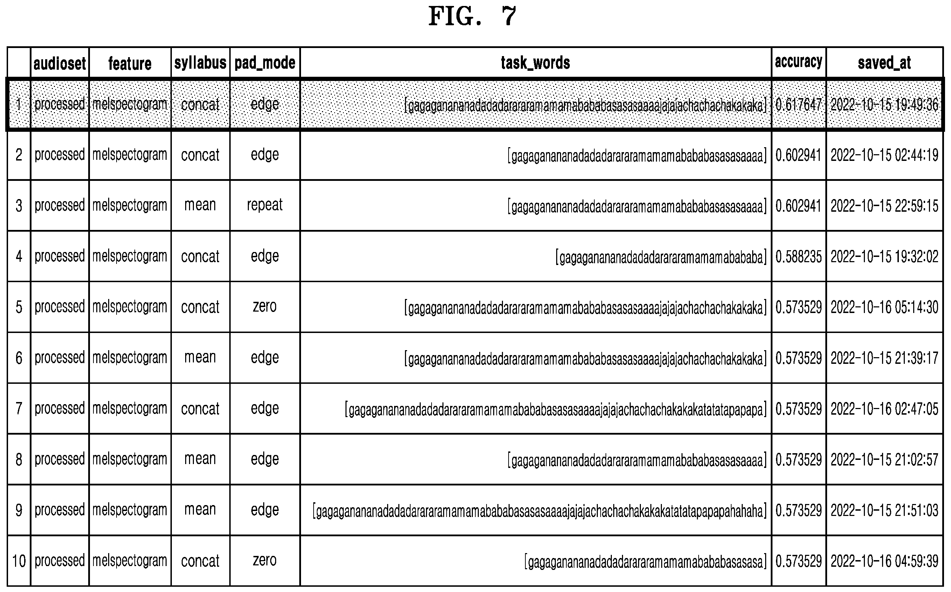

FIG. 7 is a diagram illustrating the accuracy for the first different syntactic combinations from the top first rank to the top tenth rank, based on the result of the first verification. Referring to FIG. 7, the determiner 150 may determine "[gagagananana...kakaka]," which is one of the first syntactic combinations ranked in the highest level (first rank), as the audio data for the Parkinson's disease prediction.

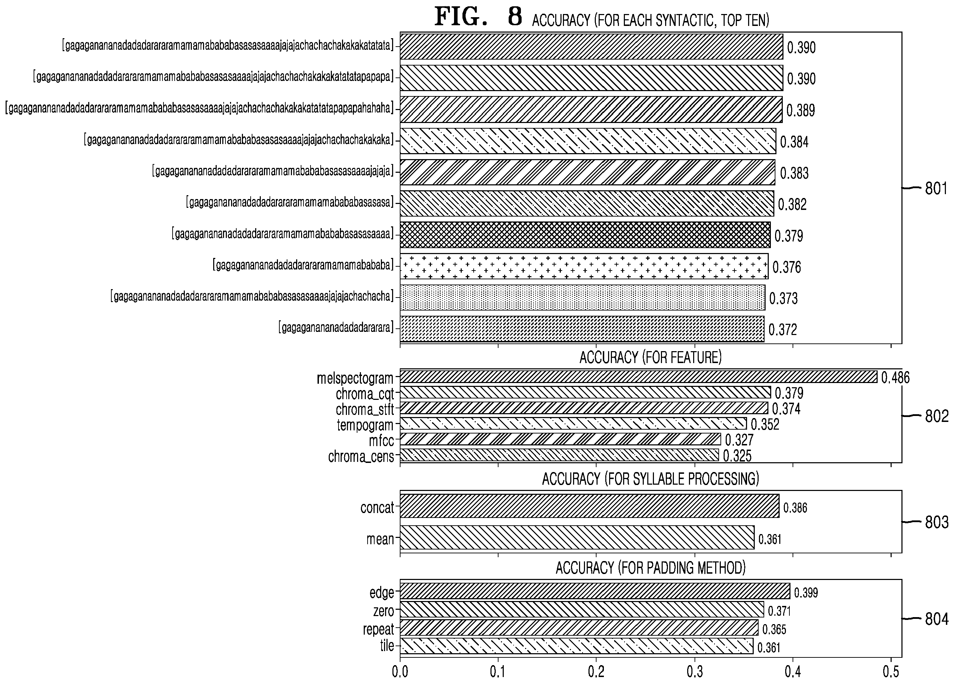

FIG. 8 is a diagram illustrating a result of comparing accuracy averages of factors included in the first preprocessing condition, based on the result of the first verification. Referring to FIG. 8, 801 is a result of comparing accuracy averages for the first different syntactic combinations. 802 is a result of comparing accuracy averages for the audio feature values for each method of extracting the audio feature values. 803 is a result of comparing accuracy averages for each syllable processing. 804 is a result of comparing accuracy averages for each type of data padding.

The determiner 150 may determine the basic vowel set ranked in the highest rank as the audio data for the Parkinson's disease prediction, based on the result of the second verification output from the verifier 140.

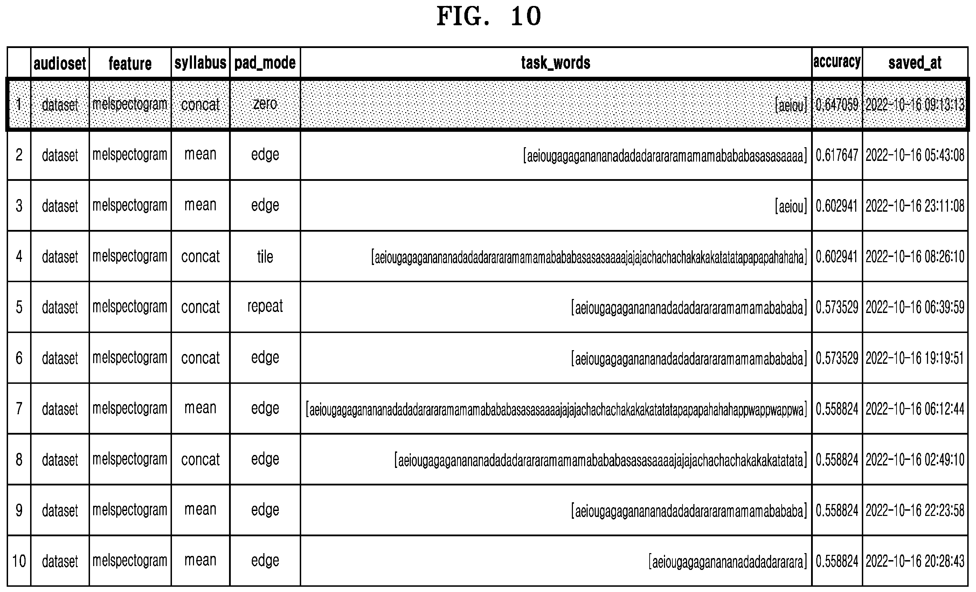

FIG. 10 is a diagram illustrating the accuracy for the second different syntactic combinations from the top first rank to the top tenth rank, based on the result of the second verification. Referring to FIG. 10, the determiner 150 may determine the basic vowel set (i.e., "[aeiou]"), which is one of the second syntactic combinations ranked in the highest rank (first rank), as the audio data for the Parkinson's disease prediction.

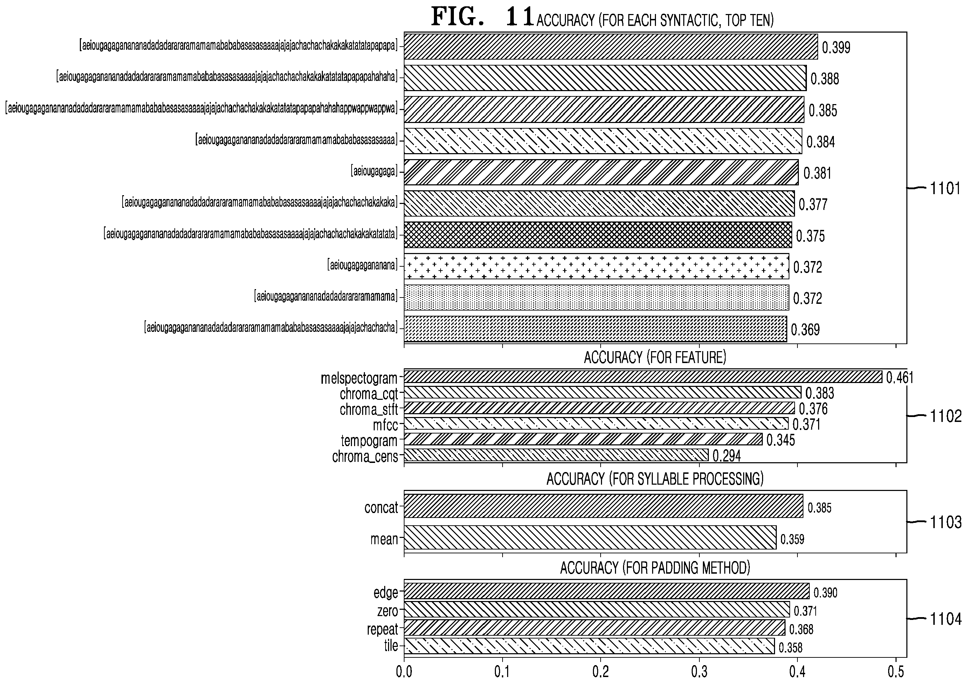

FIG. 11 is a diagram illustrating a result of comparing accuracy averages of factors included in the second preprocessing condition, based on the result of the second verification. Referring to FIG. 11, 1101 is a result of comparing accuracy averages for the second different syntactic combinations. 1102 is a result of comparing accuracy averages for the audio feature values for each method of extracting the audio feature values. 1103 is a result of comparing accuracy averages for each syllable processing. 1104 is a result of comparing accuracy averages for each type of data padding.

The determiner 150 may determine the third syntactic combination ranked in the highest rank as the audio data for the Parkinson's disease prediction, based on the result of the third verification output from the verifier 140. The third syntactic combination ranked in the highest rank may be a syntactic combination in which the third different syllables generated by combining the third and eighth consonants according to the consonant order with the certain vowel are repeated a preset number of times.

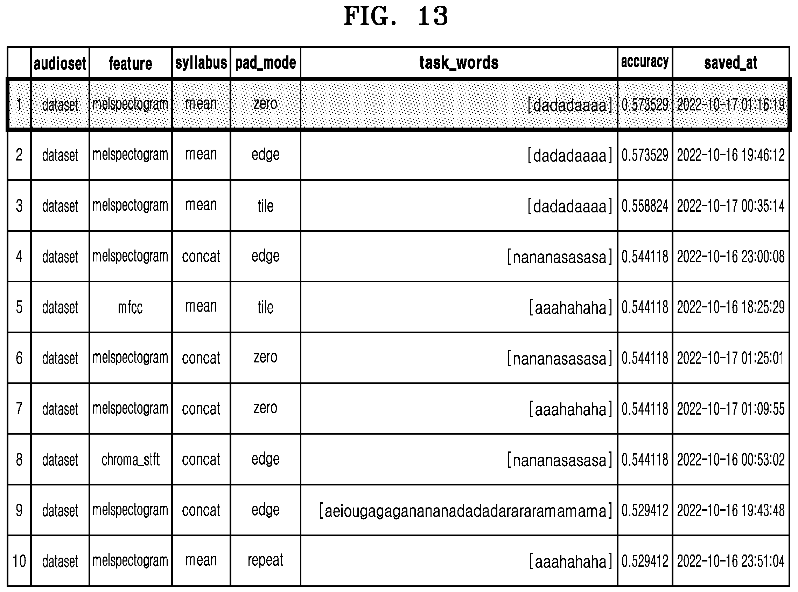

FIG. 13 is a diagram illustrating the accuracy for the third different syntactic combinations from the top first rank to the top tenth rank, based on the result of the third verification. Referring to FIG. 13, the determiner 150 may determine "[dadadaaaa]," which is one of the third syntactic combinations ranked in the highest rank (first rank), as the audio data for the Parkinson's disease prediction. In the present embodiment, the "[dadadaaaa]" ranked in the highest rank (first rank) may be one of the third syntactic combinations in which a mel-scaled spectrogram extraction method is executed as the extraction of the audio feature values included in the third preprocessing condition, mean processing is executed as the syllable processing, and zero is executed as the type of data padding.

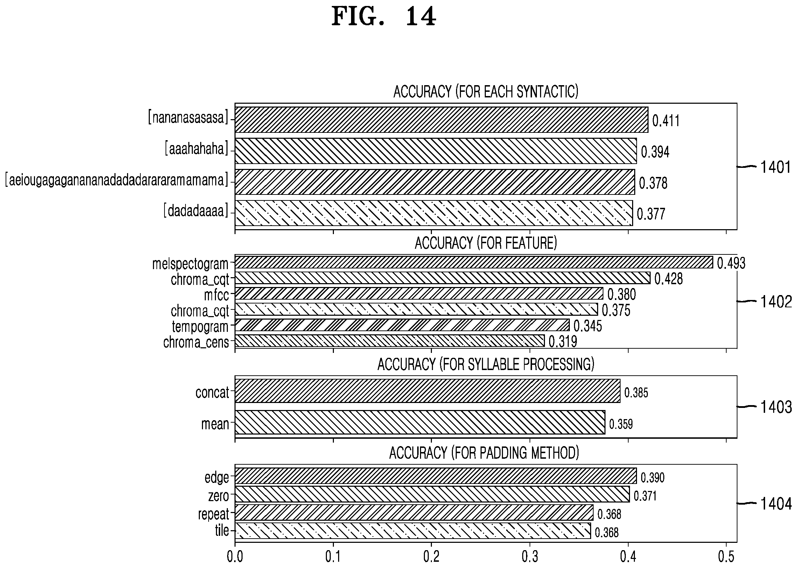

FIG. 14 is a diagram illustrating a result of comparing accuracy averages of factors included in the third preprocessing condition, based on the result of the third verification. Referring to FIG. 14, 1401 is a result of comparing accuracy averages for the third different syntactic combinations. 1402 is a result of comparing accuracy averages for the audio feature values for each method of extracting the audio feature values. 1403 is a result of comparing accuracy averages for each syllable processing. 1404 is a result of comparing accuracy averages for each type of data padding.

The determiner 150 may determine the third syntactic combination ranked in the highest rank as the audio data for the Parkinson's disease prediction, based on the result of the fourth verification output from the verifier 140. The third syntactic combination ranked in the highest rank may be a syntactic combination in which third different syllables generated by combining the eighth and fourteenth consonants according to the consonant order with the certain vowel are repeated a preset number of times.

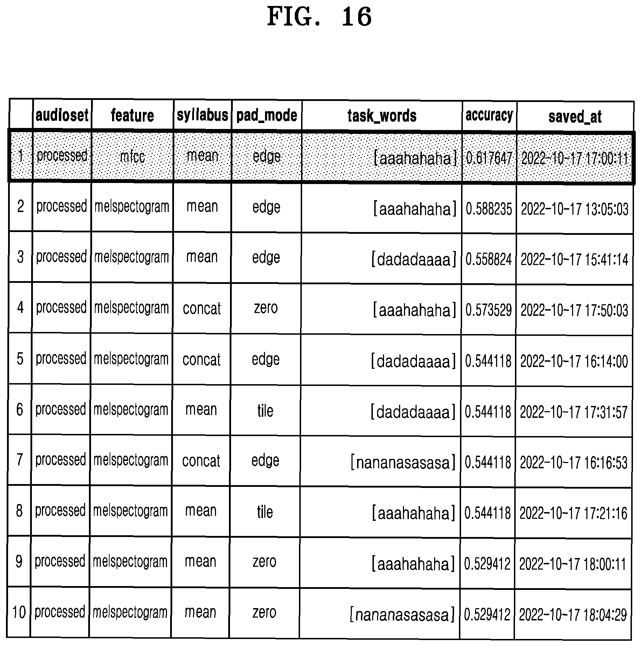

FIG. 16 is a diagram illustrating the accuracy for the third different syntactic combinations from the top first rank to the top tenth rank, based on the result of the fourth verification. Referring to FIG. 16, the determiner 150 may determine "[aaahahaha]," which is one of the third syntactic combinations ranked in the highest rank (first rank), as the audio data for the Parkinson's disease prediction. In the present embodiment, the "[aaahahaha]" ranked in the highest rank (first rank) may be one of the third syntactic combinations in which a mel-scaled spectrogram extraction method is executed as the extraction of the audio feature values included in the fourth preprocessing condition, mean processing is executed as the syllable processing, and edge is executed as the type of data padding.

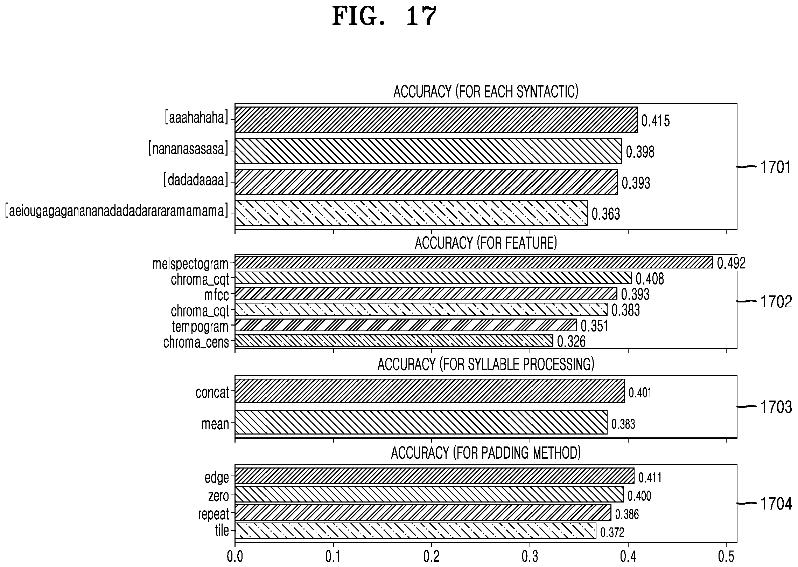

FIG. 17 is a diagram illustrating a result of comparing accuracy averages of factors included in the fourth preprocessing condition, based on the result of the fourth verification. Referring to FIG. 17, 1701 is a result of comparing accuracy averages for the third different syntactic combinations. 1702 is a result of comparing accuracy averages for the audio feature values for each method of extracting the audio feature values. 1703 is a result of comparing accuracy averages for each syllable processing. 1704 is a result of comparing accuracy averages for each type of data padding.

The determiner 150 may determine the third syntactic combination ranked in the highest rank as the audio data for the Parkinson's disease prediction, based on the result of the fifth verification output from the verifier 140. The third syntactic combination ranked in the highest rank may be a syntactic combination in which third different syllables generated by combining the second and seventh consonants according to the consonant order with the certain vowel are repeated a preset number of times.

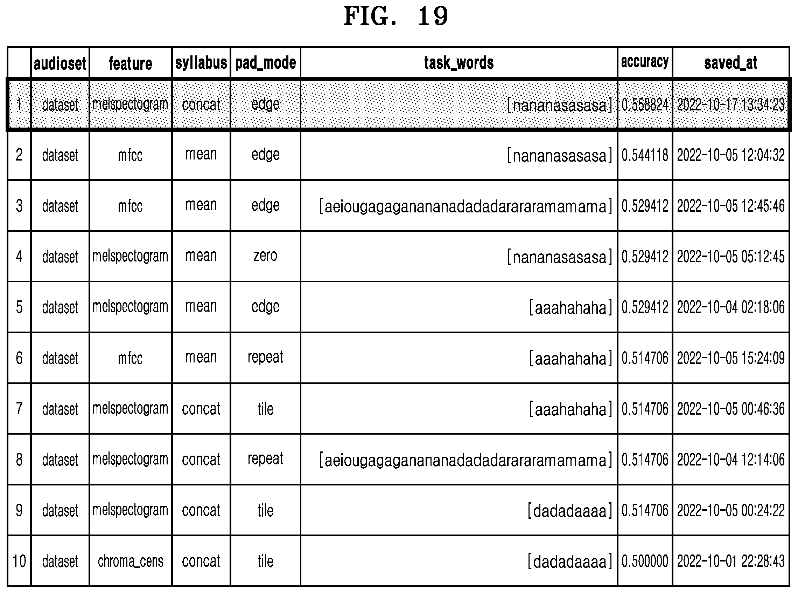

FIG. 19 is a diagram illustrating the accuracy for the third different syntactic combinations from the top first rank to the top tenth rank, based on the result of the fifth verification. Referring to FIG. 19, the determiner 150 may determine "[nananasasasa]," which is one of the third syntactic combinations ranked in the highest rank (first rank), as the audio data for the Parkinson's disease prediction. In the present embodiment, the "[nananasasasa]" ranked in the highest rank (first rank) may be one of the third syntactic combinations in which a mel-scaled spectrogram extraction method is executed as the extraction of the audio feature values included in the fifth preprocessing condition, concat processing is executed as the syllable processing, and edge is executed as the type of data padding.

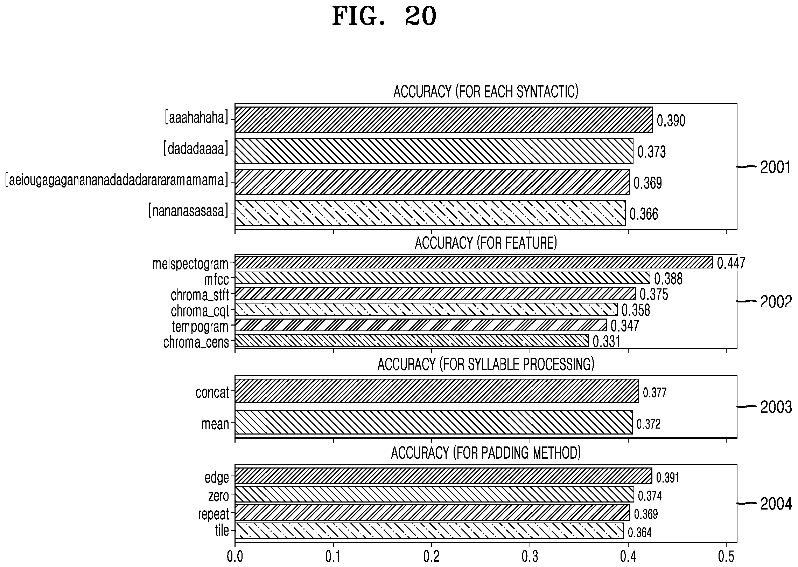

FIG. 20 is a diagram illustrating a result of comparing accuracy averages of factors included in the fifth preprocessing condition, based on the result of the fifth verification. Referring to FIG. 20, 2001 is a result of comparing accuracy averages for the third different syntactic combinations. 2002 is a result of comparing accuracy averages for the audio feature values for each method of extracting the audio feature values. 2003 is a result of comparing accuracy averages for each syllable processing. 2004 is a result of comparing accuracy averages for each type of data padding.

The determiner 150 may determine the first syntactic combination ranked in the highest rank as the audio data for the Parkinson's disease prediction, based on the result of the sixth verification output from the verifier 140. The first syntactic combination ranked in the highest rank may be a syntactic combination in which first different syllables generated by combining the first to ninth consonants according to the consonant order with the certain vowel are repeated a preset number of times.

In addition, the determiner 150 may determine the first syntactic combination ranked in the second highest rank as the audio data for the Parkinson's disease prediction. The first syntactic combination ranked in the second highest rank may be a syntactic combination in which first different syllables generated by combining the first to seventh consonants according to the consonant order with the certain vowel are repeated a preset number of times.

In addition, the determiner 150 may determine the first syntactic combination ranked in the third highest rank as the audio data for the Parkinson's disease prediction. The first syntactic combination ranked in the third highest rank may be a syntactic combination in which first different syllables generated by combining to the first to sixth consonants according to the consonant order with the certain vowel are repeated a preset number of times.

In addition, the determiner 150 may determine the first syntactic combination ranked in the fourth highest rank as the audio data for the Parkinson's disease prediction. The first syntactic combination ranked in the fourth highest rank may be a syntactic combination in which first different syllables generated by combining the first to fifth consonants according to the consonant order with the certain vowel are repeated a preset number of times.

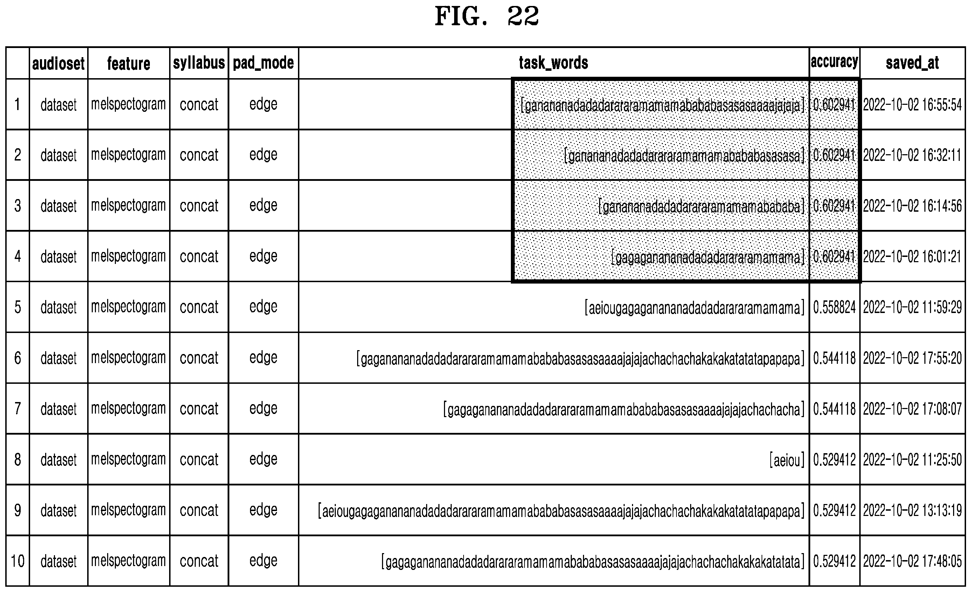

FIG. 22 is a diagram illustrating the accuracy for the first different syntactic combinations and the second different syntactic combinations from the top first rank to the top tenth rank, based on the result of the sixth verification. Referring to FIG. 22, the determiner 150 may determine "[gagagananana...jajaja]," which is one of the first syntactic combinations ranked in the highest rank (first rank), as the audio data for the Parkinson's disease prediction.

In addition, the determiner 150 may determine "[gagagananana...sasasa]," which is one of the first syntactic combinations ranked in the second highest rank (second rank), as the audio data for the Parkinson's disease prediction.

In addition, the determiner 150 may determine "[gagagananana... bababa]," which is one of the first syntactic combinations ranked in the third highest rank (third rank), as the audio data for the Parkinson's disease prediction.

In addition, the determiner 150 may determine "[gagagananana...mamama]," which is one of the first syntactic combinations ranked in the fourth highest rank (fourth rank), as the audio data for the Parkinson's disease prediction.

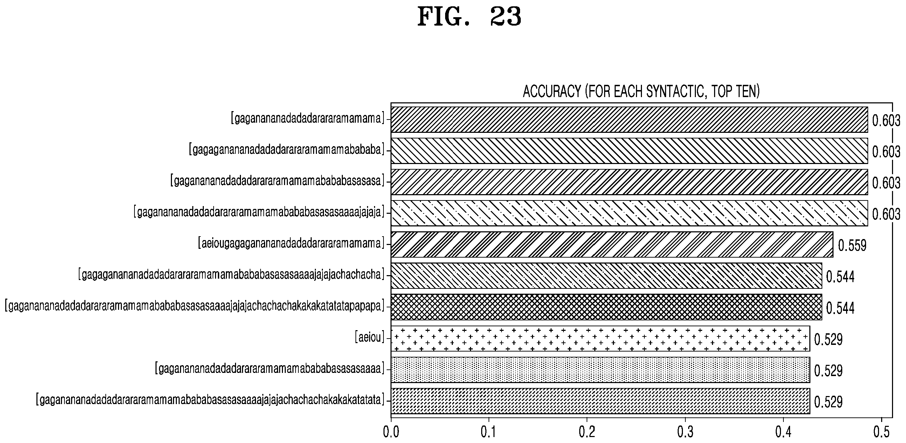

FIG. 23 illustrates a result of comparing accuracy averages for the first different syntactic combinations and the second different syntactic combinations, based on the result of the sixth verification.

The determiner 150 may determine the first syntactic combination ranked in the highest rank as the audio data for the Parkinson's disease prediction, based on the result of the seventh verification output from the verifier 140. The first syntactic combination ranked in the highest rank may be a syntactic combination in which first different syllables generated by combining the first to ninth consonants according to the consonant order with the certain vowel are repeated a preset number of times.

In addition, the determiner 150 may determine the first syntactic combination ranked in the second highest rank as the audio data for the Parkinson's disease prediction. The first syntactic combination ranked in the second highest rank may be a syntactic combination in which first different syllables generated by combining the first to seventh consonants according to the consonant order with the certain vowel are repeated a preset number of times.

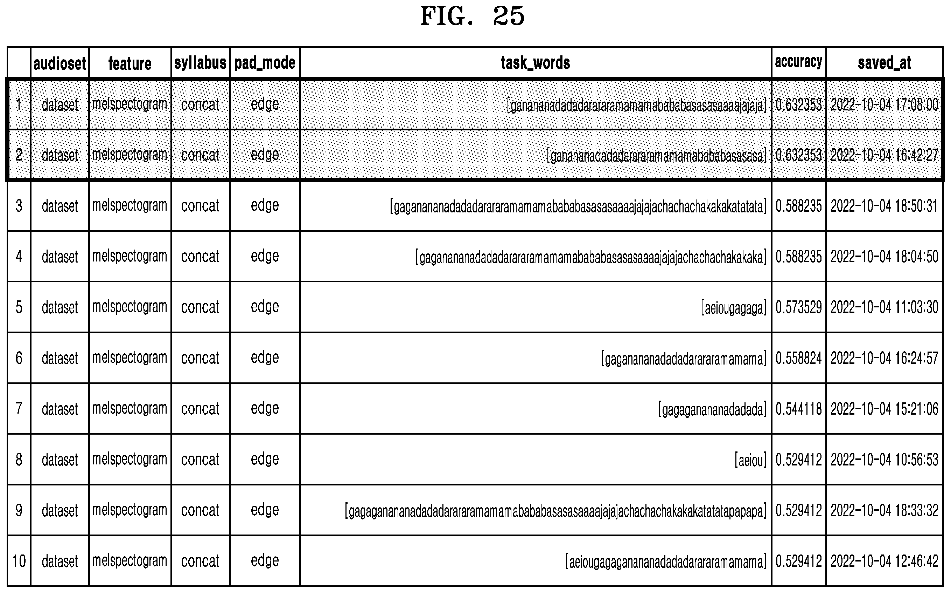

FIG. 25 is a diagram illustrating the accuracy for the first different syntactic combinations and the second different syntactic combinations from the top first rank to the top tenth rank, based on the result of the seventh verification. Referring to FIG. 25, the determiner 150 may determine "[gagagananana...jajaja]," which is one of the first syntactic combinations ranked in the highest rank (first rank), as the audio data for the Parkinson's disease prediction.

In addition, the determiner 150 may determine "[gagagananana...sasasa]," which is one of the first syntactic combinations ranked in the second highest rank (second rank), as the audio data for the Parkinson's disease prediction.

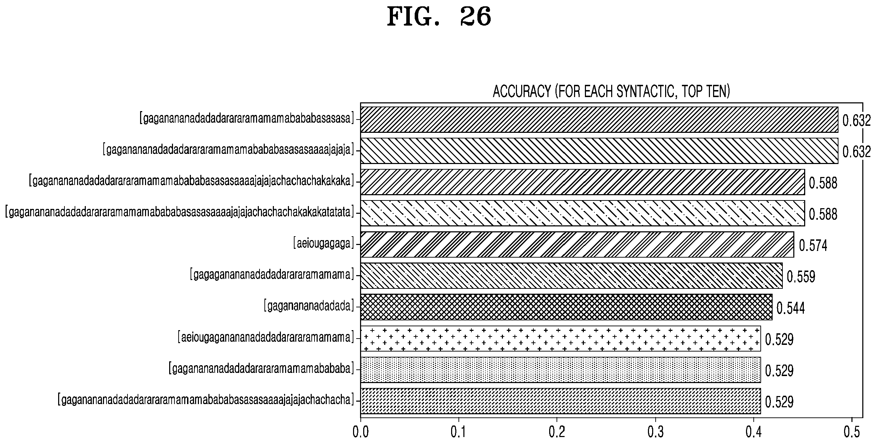

FIG. 26 illustrates a result of comparing accuracy averages for the first different syntactic combinations and the second different syntactic combinations, based on the result of the seventh verification.

The obtainer 160 may input, to the Parkinson's disease prediction model, a speaker's speech result corresponding to the audio data for the Parkinson's disease prediction determined by the determiner 150 and may obtain a Parkinson's disease prediction result for the speaker as an output of the Parkinson's disease prediction model.

The controller 170 may control overall operations of the Parkinson's disease prediction apparatus 100. The controller 170 may include any types of devices capable of processing data, such as a processor. The processor may refer to, for example, a data processing device embedded in hardware having a physically structured circuit to execute functions represented by code or instructions included in a program. Examples of the data processing device embedded in hardware may include processing devices, such as a microprocessor, a central processing unit (CPU), a processor core, a multiprocessor, an application-specific integrated circuit (ASIC), and a field programmable gate array (FPGA), but the scope of the disclosure is not limited thereto.

In an alternative embodiment, the generator 120 may generate a gender classification model. In the present embodiment, the gender classification model may be generated by training a deep neural network model pre-trained to classify a speaker's gender by using the audio data including the speaker's speech result. The deep neural network model may be a model that receives the audio data including the speaker's speech result and is trained in a supervised learning method by using training data labeled with the speaker's gender.

The generator 120 may train an initially configured deep neural network model in a supervised learning method by using the labeled training data. The initially configured deep neural network model is an initial model designed to be configured as the gender classification model, and parameter values are in a state of being set to arbitrary initial values. As the initially configured deep neural network model is trained by using the training data, the parameter values may be optimized. Therefore, the initially configured deep neural network model may be completed as the gender classification model capable of accurately classifying the speaker's gender.

The verifier 140 may verify the accuracy of the gender classification model based on the first different syntactic combinations, the second different syntactic combinations, and the third different syntactic combinations. When verifying the accuracy of the gender classification model, the verifier 140 may apply the conditions for preprocessing the audio data.

The determiner 150 may determine the third syntactic combination ranked in the highest rank as the audio data for gender classification, based on the result of verifying the accuracy of the gender classification model. The third syntactic combination ranked in the highest rank may be a syntactic combination in which third different syllables generated by combining the third and eighth consonants according to the consonant order with the certain vowel are repeated a preset number of times.

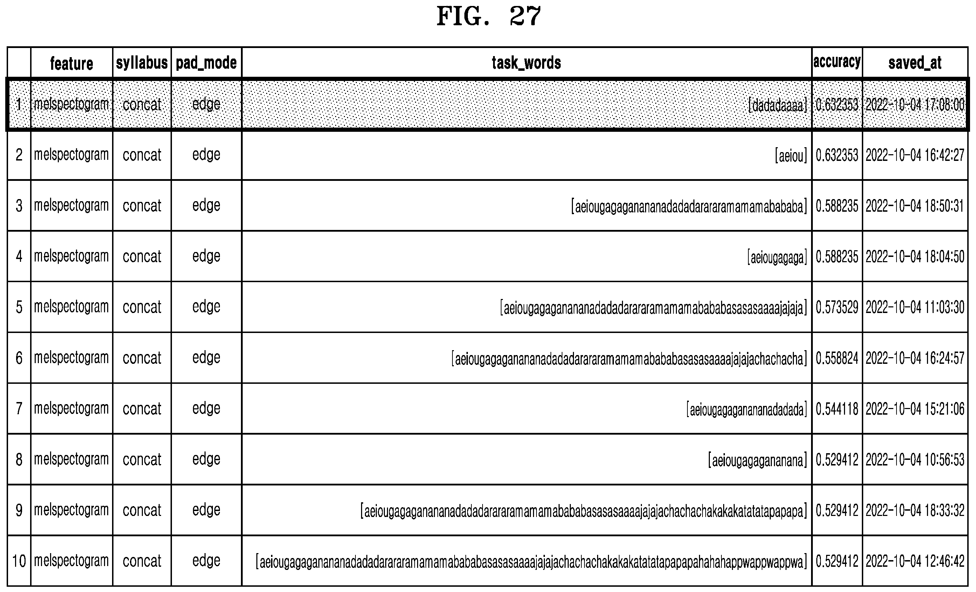

FIG. 27 is a diagram illustrating the accuracy for the first different syntactic combinations, the second different syntactic combinations, and the third different syntactic combinations from the top first rank to the top tenth rank, based on the result of verifying the gender classification model. Referring to FIG. 27, the determiner 150 may determine "[dadadaaaa]," which is one of the third syntactic combinations ranked in the highest rank (first rank), as the audio data for gender classification.

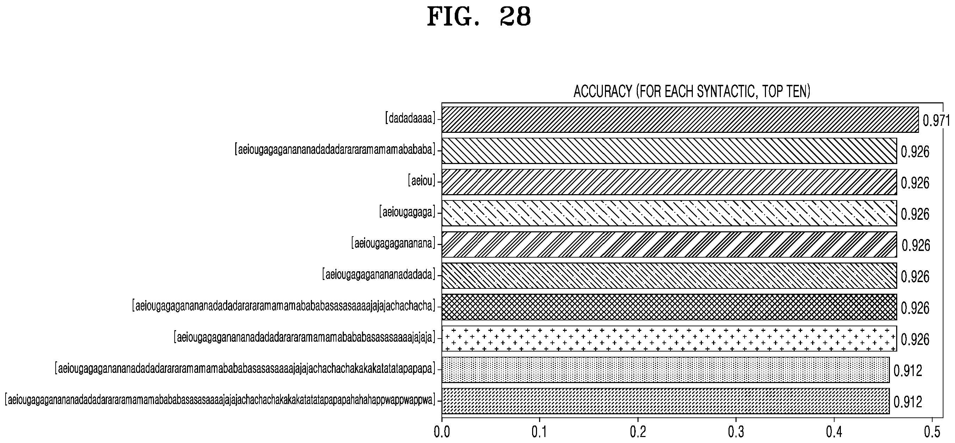

FIG. 28 illustrates a result of comparing accuracy averages for the first different syntactic combinations and the second different syntactic combinations, based on the result of verifying the accuracy of the gender classification model.

The obtainer 160 may input, to the gender classification model, a speaker's speech result corresponding to the audio data determined by the determiner 150 and may obtain a gender classification result for the speaker as an output of the gender classification model.

In an alternative embodiment, the generator 120 may generate a syntactic performance comparison model. In the present embodiment, the syntactic performance comparison model may be generated by training a deep neural network model pre-trained to output a performance comparison result for each syntactic included in the audio data by using the audio data including the speaker's speech result. The deep neural network model may be a model that receives the audio data including the speaker's speech result and is trained in a supervised learning method by using training data labeled with the performance comparison result for each syntactic.

The generator 120 may train an initially configured deep neural network model in a supervised learning method by using the labeled training data. The initially configured deep neural network model is an initial model designed to be configured as the syntactic performance comparison model, and parameter values are in a state of being set to arbitrary initial values. As the initially configured deep neural network model is trained by using the training data, the parameter values may be optimized. Therefore, the initially configured deep neural network model may be completed as the syntactic performance comparison model capable of accurately comparing performance for each syntactic.

The verifier 140 may verify the accuracy of the syntactic performance comparison model for the Parkinson's disease prediction, based on certain syntactics extracted from the audio data including the speaker's speech result. When verifying the accuracy of the syntactic performance comparison model, the verifier 140 may apply the conditions for preprocessing the audio data.

The determiner 150 may determine the syntactics ranked in a high level as the audio data for syntactic performance comparison for the Parkinson's disease prediction, based on the result of verifying the accuracy of the syntactic performance comparison model for the Parkinson's disease prediction.

The determiner 150 may determine the first syntactic ranked in the first rank as the audio data for syntactic performance comparison for the Parkinson's disease prediction, based on the result of verifying the accuracy of the syntactic performance comparison model for the Parkinson's disease prediction. The first syntactic ranked in the first rank may be a syntactic in which the first syllable generated by combining the fourth consonant according to the consonant order with the certain vowel is repeated a preset number of times.

In addition, the determiner 150 may determine the first syntactic ranked in the second rank as the audio data for syntactic performance comparison for the Parkinson's disease prediction. The first syntactic ranked in the second rank may be a syntactic in which the first syllable generated by combining the eighth consonant according to the consonant order with the certain vowel is repeated a preset number of times.

In addition, the determiner 150 may determine the basic vowel set ranked in the third rank as the audio data for syntactic performance comparison for the Parkinson's disease prediction.

In addition, the determiner 150 may determine the first syntactic ranked in the fourth rank as the audio data for syntactic performance comparison for the Parkinson's disease prediction. The first syntactic ranked in the fourth rank may be a syntactic in which the first syllable generated by combining the eleventh consonant according to the consonant order with the certain vowel is repeated a preset number of times.

In addition, the determiner 150 may determine the first syntactic ranked in the fifth rank as the audio data for syntactic performance comparison for the Parkinson's disease prediction. The first syntactic ranked in the fifth rank may be a syntactic in which the first syllables generated by combining the second consonant according to the consonant order with the certain vowel is repeated a preset number of times. In addition, the determiner 150 may determine the first syntactic ranked in the sixth rank as the audio data for syntactic performance comparison for the Parkinson's disease prediction. The first syntactic ranked in the sixth rank may be a syntactic in which the first syllable generated by combining the sixth consonant according to the consonant order with the certain vowel is repeated a preset number of times.

In addition, the determiner 150 may determine the first syntactic ranked in the seventh rank as the audio data for syntactic performance comparison for the Parkinson's disease prediction. The first syntactic ranked in the seventh rank may be a syntactic in which the first syllable generated by combining the twelfth consonant according to the consonant order with the certain vowel is repeated a preset number of times.

In addition, the determiner 150 may determine the first syntactic ranked in the eighth rank as the audio data for syntactic performance comparison for the Parkinson's disease prediction. The first syntactic ranked in the eighth rank may be a syntactic in which the first syllable generated by combining the fourteenth consonant according to the consonant order with the certain vowel is repeated a preset number of times.

In addition, the determiner 150 may determine the first syntactic ranked in the ninth rank as the audio data for syntactic performance comparison for the Parkinson's disease prediction. The first syntactic ranked in the ninth rank may be a combination of the first syllable generated by combining the thirteenth consonant according to the consonant order with the certain vowel, the first syllable generated by combining the twelfth consonant according to the consonant order with the certain vowel, and the first syllable generated by combining the eleventh consonant according to the consonant order with the certain vowel.

In addition, the determiner 150 may determine the first syntactic ranked in the tenth rank as the audio data for syntactic performance comparison for the Parkinson's disease prediction. The first syntactic ranked in the tenth rank may be a syntactic in which the first syllable generated by combining the fifth consonant according to the consonant order with the certain vowel is repeated a preset number of times.

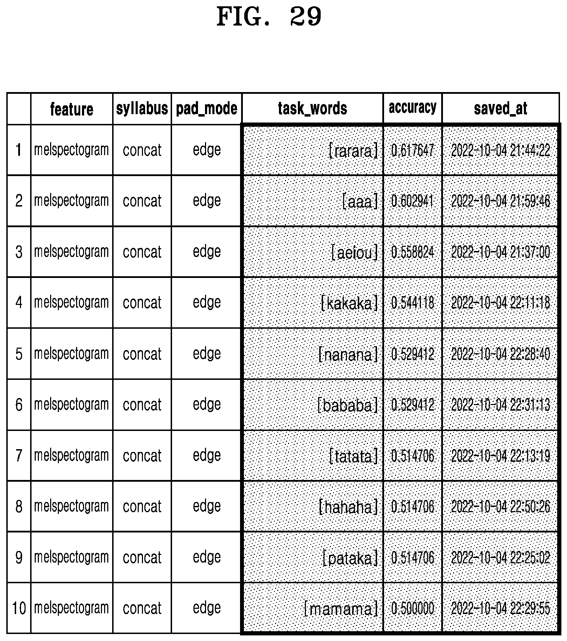

FIG. 29 is a diagram illustrating the accuracy for the first different syntactics from the top first rank to the top tenth rank, based on the result of verifying the accuracy of the syntactic performance comparison model. Referring FIG. 29, the determiner 150 may determine "[rarara]," which is one of the first syntactics ranked in the first rank, as the audio data for syntactic performance comparison. The determiner 150 may determine "[aaa]," which is one of the first syntactics ranked in the second rank, as the audio data for syntactic performance comparison. The determiner 150 may determine "[aeiou]," which is the basic vowel set ranked in the third rank, as the audio data for syntactic performance comparison. The determiner 150 may determine "[kakaka]," which is one of the first syntactics ranked in the fourth rank, as the audio data for syntactic performance comparison. The determiner 150 may determine "[nanana]," which is one of the first syntactics ranked in the fifth rank, as the audio data for syntactic performance comparison. The determiner 150 may determine "[bababa]," which is one of the first syntactics ranked in the sixth rank, as the audio data for syntactic performance comparison. The determiner 150 may determine "[tatata]," which is one of the first syntactics ranked in the seventh rank, as the audio data for syntactic performance comparison. The determiner 150 may determine "[hahaha]," which is one of the first syntactics ranked in the eighth rank, as the audio data for syntactic performance comparison. The determiner 150 may determine "[pataka]," which is one of the first syntactics ranked in the ninth rank, as the audio data for syntactic performance comparison. The determiner 150 may determine "[mamama]," which is one of the first syntactics ranked in the tenth rank, as the audio data for syntactic performance comparison.

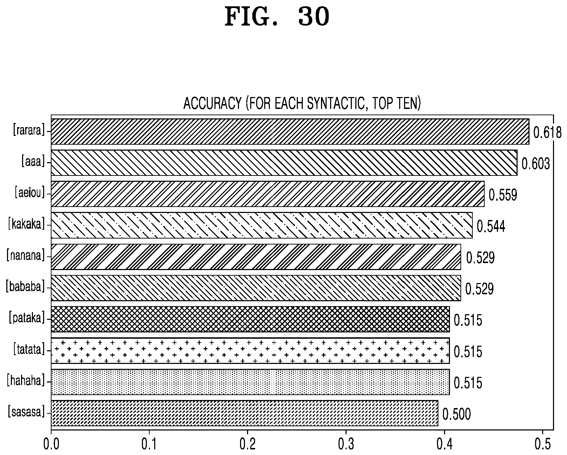

FIG. 30 illustrates a result of comparing accuracy averages for the first different syntactics and the basic vowel set, based on the result of verifying the accuracy of the syntactic performance comparison model.

The obtainer 160 may input, to the syntactic performance comparison model, a speaker's speech result corresponding to the audio data determined by the determiner 150 and may obtain a syntactic performance comparison result for the Parkinson's disease prediction for the speaker as an output of the syntactic performance comparison model.

In an alternative embodiment, the generator 120 may generate a first Parkinson's disease accuracy classification model. In the present embodiment, the first Parkinson's disease accuracy classification model may be generated by training a deep neural network model pre-trained to classify Parkinson's disease accuracy for the speaker by using the audio data including the speaker's speech result. The deep neural network model may be a model that receives the audio data including the speaker's speech result and is trained in a supervised learning method by using training data labeled with a first class and/or a second class and a third class. In the present embodiment, the first class may include a disease with the highest accuracy from among normal, Parkinson, multiple system atrophy, and cerebellar atrophy included in the Parkinson's disease-related diseases. The second class may include a disease with the second highest accuracy from among normal, Parkinson, multiple system atrophy, and cerebellar atrophy included in the Parkinson's disease-related diseases. The third class may include the remaining Parkinson's disease-related diseases excluding the first class or the second class.

The generator 120 may train an initially configured deep neural network model in a supervised learning method by using the labeled training data. The initially configured deep neural network model is an initial model designed to be configured as the first Parkinson's disease accuracy classification model, and parameter values are in a state of being set to arbitrary initial values. As the initially configured deep neural network model is trained by using the training data, the parameter values may be optimized. Therefore, the initially configured deep neural network model may be completed as the first Parkinson's disease accuracy classification model capable of accurately classifying the Parkinson's disease accuracy.

The verifier 140 may verify the accuracy of the first Parkinson's disease accuracy classification model based on the first different syntactic combinations, the second different syntactic combinations, and the third different syntactic combinations. When verifying the accuracy of the first Parkinson's disease accuracy classification model, the verifier 140 may apply the conditions for preprocessing the audio data.

The determiner 150 may determine a syntactic combination ranked in a high level as the audio data for classifying the Parkinson's disease accuracy, based on the result of verifying the accuracy of the first Parkinson's disease accuracy classification model, which is output from the verifier 140.

The determiner 150 may determine the third syntactic combination ranked in the highest rank as the audio data for classifying the Parkinson's disease accuracy, based on the result of verifying the accuracy of the first Parkinson's disease accuracy classification model, which is output from the verifier 140. The third syntactic combination ranked in the highest rank may correspond to cerebellar atrophy as the first class and the third class including the remaining Parkinson's disease-related diseases excluding cerebellar atrophy. In addition, the third syntactic combination ranked in the highest rank may be a syntactic combination in which the third different syllables generated by combining the third and eighth consonants according to the consonant order with the certain vowel are repeated a preset number of times.

In addition, the determiner 150 may determine the third syntactic combination ranked in the second highest rank as the audio data for classifying the Parkinson's disease accuracy. The third syntactic combination ranked in the second highest rank may correspond to normal as the first class and the third class including the remaining Parkinson's disease-related diseases excluding normal. In addition, the third syntactic combination ranked in the second highest rank may be a syntactic combination in which the third different syllables generated by combining the eighth and fourteenth consonants according to the consonant order with the certain vowel are repeated a preset number of times.

In addition, the determiner 150 may determine the third syntactic combination ranked in the third highest rank as the audio data for classifying the Parkinson's disease accuracy. The third syntactic combination ranked in the third highest rank may correspond to multiple system atrophy as the first class and the third class including the remaining Parkinson's disease-related diseases excluding multiple system atrophy. In addition, the third syntactic combination ranked in the third highest rank may be a syntactic combination in which the third different syllables generated by combining the second and seventh consonants according to the consonant order with the certain vowel are repeated a preset number of times.

In addition, the determiner 150 may determine the third syntactic combination ranked in the fourth highest rank as the audio data for classifying the Parkinson's disease accuracy. The third syntactic combination ranked in the fourth highest rank may correspond to Parkinson as the first class and the third class including the remaining Parkinson's disease-related diseases excluding Parkinson. In addition, the third syntactic combination ranked in the fourth highest rank may be a syntactic combination in which the third different syllables generated by combining the eighth and fourteenth consonants according to the consonant order with the certain vowel are repeated a preset number of times.

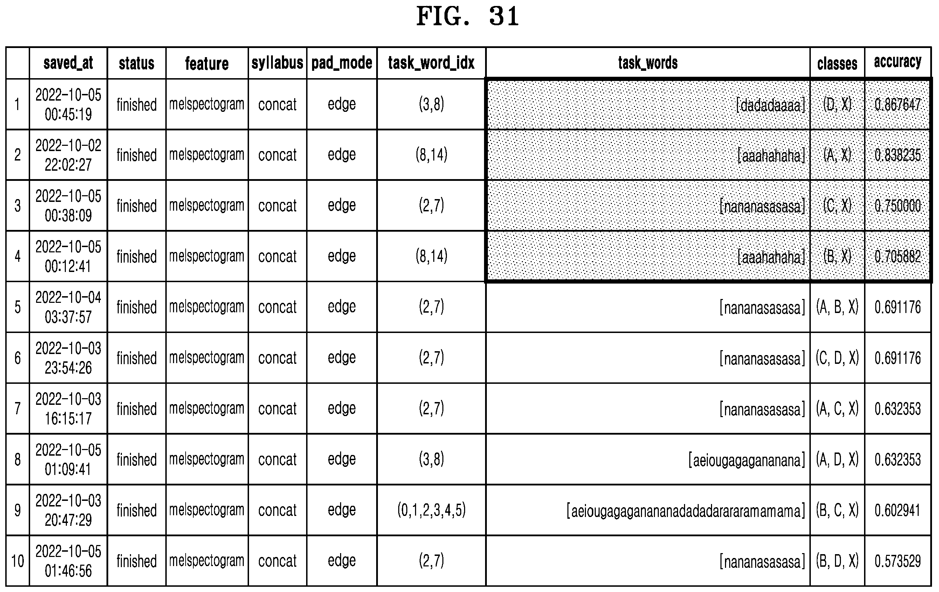

FIG. 31 is a diagram illustrating the accuracy for the first different syntactic combinations, the second different syntactic combinations, and the third different syntactic combinations from the top first rank to the top tenth rank, based on the result of verifying the accuracy of the first Parkinson's disease accuracy classification model.

Referring to FIG. 31, the determiner 150 may determine "[dadadaaaa]," which is one of the third syntactic combinations ranked in the first rank, as the audio data for classifying the first Parkinson's disease accuracy. The "[dadadaaaa]" ranked in the first rank may be a syntactic combination capable of distinguishing between cerebellar atrophy (D) as the first class and normal, Parkinson, and multiple system atrophy as the third class, which are the remaining Parkinson's disease-related diseases (X) excluding cerebellar atrophy, with the highest accuracy.

In addition, the determiner 150 may determine "[aaahahaha]," which is one of the third syntactic combinations ranked in the second rank, as the audio data for classifying the first Parkinson's disease accuracy. The "[aaahahaha]" ranked in the second rank may be a syntactic combination capable of distinguishing between normal (A) as the first class and Parkinson, multiple system atrophy, and cerebellar atrophy as the third class, which are the remaining Parkinson's disease-related diseases (X) excluding normal (A), with the highest accuracy.

In addition, the determiner 150 may determine "[nananasasasa]," which is one of the third syntactic combinations ranked in the third rank, as the audio data for classifying the first Parkinson's disease accuracy. The "[nananasasasa]" ranked in the third rank may be a syntactic combination capable of distinguishing between multiple system atrophy (C) as the first class and normal, Parkinson, and cerebellar atrophy as the third class, which are the remaining Parkinson's disease-related diseases (X) excluding multiple system atrophy, with the highest accuracy.

In addition, the determiner 150 may determine "[aaahahaha]," which is one of the third syntactic combinations ranked in the fourth rank, as the audio data for classifying the first Parkinson's disease accuracy. The "[aaahahaha]" ranked in the fourth rank may be a syntactic combination capable of distinguishing between Parkinson (C) as the first class and normal, multiple system atrophy, and cerebellar atrophy as the third class, which are the Parkinson's disease-related diseases (X) excluding Parkinson (C), with the highest accuracy.

The obtainer 160 may input, to the first Parkinson's disease accuracy classification model, a speaker's speech result corresponding to the audio data determined by the determiner 150 and may obtain a first Parkinson's disease accuracy classification result for the speaker as an output of the first Parkinson's disease accuracy classification model.

In an alternative embodiment, the generator 120 may generate a second Parkinson's disease accuracy classification model. In the present embodiment, the second Parkinson's disease accuracy classification model may be generated by training a deep neural network model pre-trained to classify Parkinson's disease accuracy for the speaker by using the audio data including the speaker's speech result. The deep neural network model may be a model that receives the audio data including the speaker's speech result and is trained in a supervised learning method by using training data labeled with two or more of a fourth class, a fifth class, and a sixth class. In the present embodiment, the fourth class may include a disease with the highest accuracy from among normal, Parkinson, multiple system atrophy, and cerebellar atrophy included in the Parkinson's disease-related diseases. The fifth class may include a disease with higher accuracy from among the remaining three Parkinson's disease-related diseases excluding the fourth class. The sixth class may include a disease with the highest accuracy from among the remaining two Parkinson's disease-related diseases excluding the fourth class and the fifth class.

The generator 120 may train an initially configured deep neural network model in a supervised learning method by using the labeled training data. The initially configured deep neural network model is an initial model designed to be configured as the second Parkinson's disease accuracy classification model, and parameter values are in a state of being set to arbitrary initial values. As the initially configured deep neural network model is trained by using the training data, the parameter values may be optimized. Therefore, the initially configured deep neural network model may be completed as the second Parkinson's disease accuracy classification model capable of accurately classifying the Parkinson's disease accuracy.

The verifier 140 may verify the accuracy of the second Parkinson's disease accuracy classification model based on the first different syntactic combinations, the second different syntactic combinations, and the third different syntactic combinations. When verifying the accuracy of the second Parkinson's disease accuracy classification model, the verifier 140 may apply the conditions for preprocessing the audio data.

The determiner 150 may determine a syntactic combination ranked in a high rank as the audio data for classifying the Parkinson's disease accuracy, based on the result of verifying the accuracy of the second Parkinson's disease accuracy classification model.

The determiner 150 may determine the first syntactic combination ranked in the first rank as the audio data for classifying the Parkinson's disease accuracy, based on the result of verifying the accuracy of the second Parkinson's disease accuracy classification model. The first syntactic combination ranked in the first rank may correspond to normal as the fourth class and cerebellar atrophy as the fifth class.

In addition, the first syntactic combination ranked in the second rank may be a syntactic combination in which the first syllables generated by In addition, the first syntactic combination ranked in the first rank may be a syntactic combination in which the first syllables generated by combining the first to seventh consonants according to the consonant order with the certain vowel are repeated a preset number of times. In addition, the determiner 150 may determine the first syntactic combination ranked in the second rank as the audio data for classifying the Parkinson's disease accuracy. The first syntactic combination ranked in the second rank may correspond to normal as the fourth class and multiple system atrophy as the fifth class.

In addition, the first syntactic combination ranked in the second rank may be a syntactic combination in which the first syllables generated by combining the first to seventh consonants according to the consonant order with the certain vowel are repeated a preset number of times. In addition, the determiner 150 may determine the first syntactic combination ranked in the third rank as the audio data for classifying the Parkinson's disease accuracy. The first syntactic combination ranked in the third rank may correspond to multiple system atrophy as the fourth class and cerebellar atrophy as the fifth class.

In addition, the first syntactic combination ranked in the third rank may be a syntactic combination in which the first syllables generated by combining the first to seventh consonants according to the consonant order with the certain vowel are repeated a preset number of times. In addition, the determiner 150 may determine the first syntactic combination ranked in the fourth rank as the audio data for classifying the Parkinson's disease accuracy. The first syntactic combination ranked in the fourth rank may correspond to Parkinson as the fourth class and cerebellar atrophy as the fifth class.

In addition, the first syntactic combination ranked in the fourth rank may be a syntactic combination in which the first syllables generated by combining the first to seventh consonants according to the consonant order with the certain vowel are repeated a preset number of times. In addition, the determiner 150 may determine the first syntactic combination ranked in the fifth rank as the audio data for classifying the Parkinson's disease accuracy. The first syntactic combination ranked in the fifth rank may correspond to normal as the fourth class, multiple system atrophy as the fifth class, and cerebellar atrophy as the sixth class. In addition, the first syntactic combination ranked in the fifth rank may be a syntactic combination in which the first syllables generated by combining the first to seventh consonants according to the consonant order with the certain vowel are repeated a preset number of times.

In addition, the determiner 150 may determine the first syntactic combination ranked in the sixth rank as the audio data for classifying the Parkinson's disease accuracy. The first syntactic combination ranked in the sixth rank may correspond to normal as the fourth class and Parkinson as the fifth class. In addition, the first syntactic combination ranked in the sixth rank may be a syntactic combination in which the first syllables generated by combining the first to ninth consonants according to the consonant order with the certain vowel are repeated a preset number of times.

In addition, the determiner 150 may determine the first syntactic combination ranked in the seventh rank as the audio data for classifying the Parkinson's disease accuracy. The first syntactic combination ranked in the seventh rank may correspond to normal as the fourth class, Parkinson as the fifth class, and cerebellar atrophy as the sixth class. In addition, the first syntactic combination ranked in the seventh rank may be a syntactic combination in which the first syllables generated by combining the first to seventh consonants according to the consonant order with the certain vowel are repeated a preset number of times.

In addition, the determiner 150 may determine the first syntactic combination ranked in the eighth rank as the audio data for classifying the Parkinson's disease accuracy. The first syntactic combination ranked in the eighth rank may correspond to Parkinson as the fourth class and multiple system atrophy as the fifth class. In addition, the first syntactic combination ranked in the eighth rank may be a syntactic combination in which the first syllables generated by combining the first to ninth consonants according to the consonant order with the certain vowel are repeated a preset number of times.

In addition, the determiner 150 may determine the first syntactic combination ranked in the ninth rank as the audio data for classifying the Parkinson's disease accuracy. The first syntactic combination ranked in the ninth rank may correspond to normal as the fourth class, Parkinson as the fifth class, and multiple system atrophy as the sixth class.

In addition, the first syntactic combination ranked in the ninth rank may be a syntactic combination in which the first syllables generated by combining the first to seventh consonants according to the consonant order with the certain vowel are repeated a preset number of times. In addition, the determiner 150 may determine the first syntactic combination ranked in the tenth rank as the audio data for classifying the Parkinson's disease accuracy. The first syntactic combination ranked in the tenth rank may correspond to Parkinson as the fourth class, multiple system atrophy as the fifth class, and cerebellar atrophy as the sixth class. In addition, the first syntactic combination ranked in the tenth rank may be a syntactic combination in which the first syllables generated by combining the first to seventh consonants according to the consonant order with the certain vowel are repeated a preset number of times.

FIG. 32 is a diagram illustrating the accuracy for the first different syntactic combinations, the second different syntactic combinations, and the third different syntactic combinations from the top first rank to the top tenth rank, based on the result of verifying the second Parkinson's disease accuracy classification model.

Referring to FIG. 32, the determiner 150 may determine "[gagagananana...sasasa]," which is one of the first syntactic combinations ranked in the first rank, as the audio data for classifying the second Parkinson's disease accuracy. The "[gagagananana...sasasa]" ranked in the first rank may be a syntactic combination capable of distinguishing between normal (A) as the fourth class and cerebellar atrophy (D) as the fifth class with the highest accuracy.

In addition, the determiner 150 may determine "[gagagananana...sasasa]," which is one of the first syntactic combinations ranked in the second rank, as the audio data for classifying the second Parkinson's disease accuracy. The "[gagagananana...sasasa]" ranked in the second rank may be a syntactic combination capable of distinguishing between normal (A) as the fourth class and multiple system atrophy (C) as the fifth class with the highest accuracy.

In addition, the determiner 150 may determine "[gagagananana...sasasa]," which is one of the first syntactic combinations ranked in the third rank, as the audio data for classifying the second Parkinson's disease accuracy. The "[gagagananana...sasasa]" ranked in the third rank may be a syntactic combination capable of distinguishing between multiple system atrophy (C) as the fourth class and cerebellar atrophy (D) as the fifth class with the highest accuracy.

In addition, the determiner 150 may determine "[gagagananana...sasasa]," which is one of the first syntactic combinations ranked in the fourth rank, as the audio data for classifying the second Parkinson's disease accuracy. The "[gagagananana...sasasa]" ranked in the fourth rank may be a syntactic combination capable of distinguishing between Parkinson (B) as the fourth class and cerebellar atrophy (D) as the fifth class with the highest accuracy.

In addition, the determiner 150 may determine "[gagagananana...sasasa]," which is one of the first syntactic combinations ranked in the fifth rank, as the audio data for classifying the second Parkinson's disease accuracy. The "[gagagananana...sasasa]" ranked in the fifth rank may be a syntactic combination capable of distinguishing between normal (A) as the fourth class, multiple system atrophy (C) as the fifth class, and cerebellar atrophy (D) as the sixth class with the highest accuracy.

In addition, the determiner 150 may determine "[gagagananana...jajaja]," which is one of the first syntactic combinations ranked in the sixth rank, as the audio data for classifying the second Parkinson's disease accuracy. The "[gagagananana...jajaja]" ranked in the sixth rank may be a syntactic combination capable of distinguishing between normal (A) as the fourth class and Parkinson (B) as the fifth class with the highest accuracy.

In addition, the determiner 150 may determine "[gagagananana...sasasa]," which is one of the first syntactic combinations ranked in the seventh rank, as the audio data for classifying the second Parkinson's disease accuracy. The "[gagagananana...sasasa]" ranked in the seventh rank may be a syntactic combination capable of distinguishing between normal (A) as the fourth class, Parkinson (B) as the fifth class, and cerebellar atrophy (D) as the sixth class with the highest accuracy.

In addition, the determiner 150 may determine "[gagagananana...jajaja]," which is one of the first syntactic combinations ranked in the eighth rank, as the audio data for classifying the second Parkinson's disease accuracy. The "[gagagananana...jajaja]" ranked in the eighth rank may be a syntactic combination capable of distinguishing between Parkinson (B) as the fourth class and multiple system atrophy (C) as the fifth class with the highest accuracy.

In addition, the determiner 150 may determine "[gagagananana...sasasa]," which is one of the first syntactic combinations ranked in the ninth rank, as the audio data for classifying the second Parkinson's disease accuracy. The "[gagagananana...sasasa]" ranked in the ninth rank may be a syntactic combination capable of distinguishing between normal (A) as the fourth class, Parkinson (B) as the fifth class, and multiple system atrophy (C) as the sixth class with the highest accuracy.

In addition, the determiner 150 may determine "[gagagananana...sasasa]," which is one of the first syntactic combinations ranked in the tenth rank, as the audio data for classifying the second Parkinson's disease accuracy. The "[gagagananana...sasasa]" ranked in the tenth rank may be a syntactic combination capable of distinguishing between Parkinson (B) as the fourth class, multiple system atrophy (C) as the fifth class, and cerebellar atrophy (D) as the sixth class with the highest accuracy.

The obtainer 160 may input, to the second Parkinson's disease accuracy classification model, a speaker's speech result corresponding to the audio data determined by the determiner 150 and may obtain a second Parkinson's disease accuracy classification result for the speaker as an output of the second Parkinson's disease accuracy classification model.

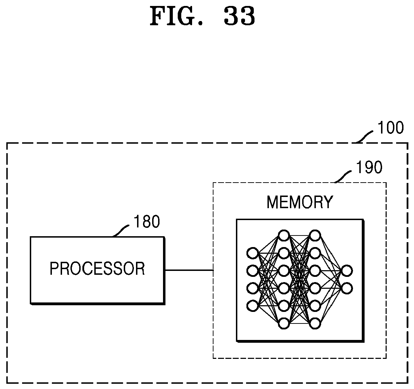

FIG. 33 is a schematic block diagram for describing a configuration of a Parkinson's disease prediction apparatus 100 according to another embodiment. In the following description, a redundant description as provided above with reference to FIGS. 1 to 32 is omitted. Referring to FIG. 33, the Parkinson's disease prediction apparatus 100 according to another embodiment may include a processor 180 and a memory 190.

In the present embodiment, the processor 180 may execute the functions of the collector 110, the generator 120, the extractor 130, the verifier 140, the determiner 150, the obtainer 160, and the controller 170 illustrated in FIG. 1.

The processor 180 may control overall operations of the Parkinson's disease prediction apparatus 100. The processor 180 may refer to, for example, a data processing device embedded in hardware having a physically structured circuit to execute functions represented by code or instructions included in a program. Examples of the data processing device embedded in hardware may include processing devices, such as a microprocessor, a CPU, a processor core, a multiprocessor, an ASIC, and an FPGA, but the scope of the disclosure is not limited thereto.

The memory 190 may be operatively connected to the processor 180 and may store at least one code associated with the operation performed by the processor 180.

In addition, the memory 190 may temporarily or permanently store data processed by the processor 180. The memory 190 may include a magnetic storage medium or a flash storage medium, but the scope of the disclosure is not limited thereto. The memory 190 may include internal memory and/or external memory. The memory 190 may include volatile memory such as dynamic random access memory (DRAM), stack random access memory (SRAM), or synchronous dynamic random access memory (SDRAM), nonvolatile memory such as one time programmable read-only memory (OTPROM), programmable read-only memory (PROM), erasable programmable read-only memory (EPROM), electrically erasable programmable read-only memory (EEPROM), mask ROM, flash ROM, NAND flash memory, or NOR flash memory, flash drive such as solid state drive (SSD), CompactFlash (CF) card, secure digital (SD) card, micro-SD card, mini-SD card, xD card, or memory stick, or storage devices such as hard disk drive (HDD).

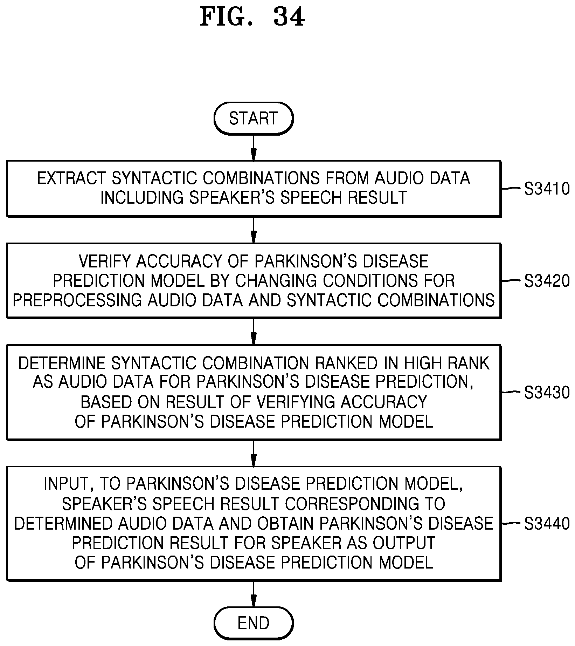

FIG. 34 is a flowchart for describing a Parkinson's disease prediction method according to the present embodiment. In the following description, a redundant description as provided above with reference to FIGS. 1 to 33 is omitted. In the following description, it is assumed that the Parkinson's disease prediction method according to the present embodiment is performed by the processor 180 of the Parkinson's disease prediction apparatus 100 with the help of peripheral components.

Referring to FIG. 34, in operation S3410, the processor 180 may extract syntactic combinations from audio data including a speaker's speech result.

The processor 180 may extract syntactic combinations including a plurality of first different syntactic combinations, a plurality of second different syntactic combinations, or a plurality of third different syntactic combinations. The first different syntactic combinations may include at least one first syllable generated by combining a certain consonant with a certain vowel from the audio data. The certain consonant included in the first different syntactic combinations may be combined with the certain vowel in the consonant order according to the language regulation, and the certain vowel included in the first different syntactic combinations may be determined as a single vowel. The first syntactic combination in which the first syllable generated by combining the n^{th} to (n+k)^{th} consonants according to the consonant order with the certain vowel is repeated a preset number of times may be extracted from the audio data. Here, n may include a natural number and k may include 0 and a natural number. The second different syntactic combinations may be a combination of one or more of a basic vowel set, a second syllable obtained by combining a certain double consonant with a certain vowel, and a first syntactic combination. The third different syntactic combinations may include at least one third syllable obtained by combining an arbitrary consonant with an arbitrary vowel, and one of the second different syntactic combinations.

In the present embodiment, the processor 180 may generate a Parkinson's disease prediction model before extracting the syntactic combination. In the present embodiment, the Parkinson's disease prediction model may be generated by training a deep neural network model pre-trained to predict Parkinson's disease for the speaker by using the audio data including the speaker's speech result. The deep neural network model may be a model that receives the audio data including the speaker's speech result and is trained in a supervised learning method by using training data labeled with one of normal, Parkinson, multiple system atrophy, and cerebellar atrophy included in the Parkinson's disease-related diseases.

In operation S3420, the processor 180 may verify the accuracy of the Parkinson's disease prediction model by changing the conditions for preprocessing the audio data and the syntactic combinations.

The processor 180 may execute a first verification to verify the accuracy of the Parkinson's disease prediction model based on a first preprocessing condition for preprocessing audio data and first different syntactic combinations. In the present embodiment, the first preprocessing condition may include a condition for executing second acoustic preprocessing, executing data padding including edge, repeat, tile, and zero, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64.

The processor 180 may execute a second verification to verify the accuracy of the Parkinson's disease prediction model based on a second preprocessing condition for preprocessing audio data and second different syntactic combinations. In the present embodiment, the second preprocessing condition may include a condition for executing first acoustic preprocessing, executing data padding including edge, repeat, tile, and zero, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64.

The processor 180 may execute a third verification to verify the accuracy of the Parkinson's disease prediction model based on a third preprocessing condition for preprocessing audio data and third different syntactic combinations. In the present embodiment, the third preprocessing condition may include a condition for executing first acoustic preprocessing, executing data padding including edge, repeat, tile, and zero, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64.

The processor 180 may execute a fourth verification to verify the accuracy of the Parkinson's disease prediction model based on a fourth preprocessing condition for preprocessing audio data and third different syntactic combinations. In the present embodiment, the fourth preprocessing condition may include a condition for executing second acoustic preprocessing, executing data padding including edge, repeat, tile, and zero, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64.

The processor 180 may execute a fifth verification to verify the accuracy of the Parkinson's disease prediction model based on a fifth preprocessing condition for preprocessing audio data and third different syntactic combinations. In the present embodiment, the fifth preprocessing condition may include a condition for executing first acoustic preprocessing, executing data padding including edge, repeat, tile, and zero, executing statistical features including a volume, a magnitude, a maximum amplitude, a length, and an average syntactic length for each speaker, executing data segmentation to segment a train set, a validation set, and a test set into 64:16:20, executing data imbalance compensation including an SMOTE algorithm and a class weight-balancing algorithm, executing scaling including standardization, executing outlier non-removal, and executing a floating point format including float64.

The processor 180 may execute a sixth verification to verify the accuracy of the Parkinson's disease prediction model based on a sixth preprocessing condition for preprocessing audio data, first different syntactic combinations, and second different syntactic combinations. In the present embodiment, the sixth preprocessing condition may include a condition for executing first acoustic preprocessing, executing data padding including edge, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64.

The processor 180 may perform a seventh verification to verify the accuracy of the Parkinson's disease prediction model based on a seventh preprocessing condition for preprocessing audio data, first different syntactic combinations, and second different syntactic combinations. In the present embodiment, the seventh preprocessing condition may include a condition for executing second acoustic preprocessing, executing data padding including edge, executing statistical features including a gender, a volume, a magnitude, a maximum amplitude, a length, and an average syntactic length for each speaker, executing data segmentation to segment a train set, a validation set, and a test set into 64:16:20, executing data imbalance compensation including an SMOTE-NC algorithm, executing scaling including standardization, executing outlier removal including isolation forest, and executing a floating point format including float64.

In operation S3430, the processor 180 may determine a syntactic combination ranked in a high rank as the audio data for the Parkinson's disease prediction, based on the result of verifying the accuracy of the Parkinson's disease prediction model.

The processor 180 may determine the first syntactic combination ranked in the highest rank as the audio data for the Parkinson's disease prediction, based on the result of the first verification for the accuracy of the Parkinson's disease prediction model. The first syntactic combination ranked in the highest rank may be a syntactic combination in which the first syllables generated by combining the first to eleventh consonants according to the consonant order with the certain vowel are repeated a preset number of times.

The processor 180 may determine the basic vowel set ranked in the highest rank as the audio data for the Parkinson's disease prediction, based on the result of the second verification for the accuracy of the Parkinson's disease prediction model.

The processor 180 may determine a third syntactic combination ranked in a highest rank as the audio data for the Parkinson's disease prediction, based on the result of the third verification for the accuracy of the Parkinson's disease prediction model. The third syntactic combination ranked in the highest rank may be a syntactic combination in which the third different syllables generated by combining the third and eighth consonants according to the consonant order with the certain vowel are repeated a preset number of times.

The processor 180 may determine the third syntactic combination ranked in the highest rank as the audio data for the Parkinson's disease prediction, based on the result of the fourth verification for the accuracy of the Parkinson's disease prediction model. The third syntactic combination ranked in the highest rank may be a syntactic combination in which the third different syllables generated by combining the eighth and fourteenth consonants according to the consonant order with the certain vowel are repeated a preset number of times.

The processor 180 may determine the third syntactic combination ranked in the highest rank as the audio data for the Parkinson's disease prediction, based on the result of the fifth verification for the accuracy of the Parkinson's disease prediction model. The third syntactic combination ranked in the highest rank may be a syntactic combination in which the third different syllables generated by combining the second and seventh consonants according to the consonant order with the certain vowel are repeated a preset number of times.

The processor 180 may determine the first syntactic combination ranked in the highest rank as audio data for the Parkinson's disease prediction, based on the result of the sixth verification for the accuracy of the Parkinson's disease prediction model. The first syntactic combination ranked in the highest rank may be a syntactic combination in which the first different syllables generated by combining the first to ninth consonants according to the consonant order with the certain vowel are repeated a preset number of times. The processor 180 may determine the first syntactic combination ranked in the second highest rank as the audio data for the Parkinson's disease prediction. The first syntactic combination ranked in the second highest rank may be a syntactic combination in which the first different syllables generated by combining the first to seventh consonants according to the consonant order with the certain vowel are repeated a preset number of times. The processor 180 may determine the first syntactic combination ranked in the third highest rank as the audio data for the Parkinson's disease prediction. The first syntactic combination ranked in the third highest rank may be a syntactic combination in which the first different syllables generated by combining the first to sixth consonants according to the consonant order with the certain vowel are repeated a preset number of times. The processor 180 may determine the first syntactic combination ranked in the fourth highest rank as the audio data for the Parkinson's disease prediction. The first syntactic combination ranked in the fourth highest rank may be a syntactic combination in which the first different syllables generated by combining the first to fifth consonants according to the consonant order with the certain vowel are repeated a preset number of times.

The processor 180 may determine the first syntactic combination ranked in the highest rank as the audio data for the Parkinson's disease prediction, based on the result of the seventh verification for the accuracy of the Parkinson's disease prediction model. The first syntactic combination ranked in the highest rank may be a syntactic combination in which the first different syllables generated by combining the first to ninth consonants according to the consonant order with the certain vowel are repeated a preset number of times. The processor 180 may determine the first syntactic combination ranked in the second highest rank as the audio data for the Parkinson's disease prediction. The first syntactic combination ranked in the second highest rank may be a syntactic combination in which the first different syllables generated by combining the first to seventh consonants according to the consonant order with the certain vowel are repeated a preset number of times.

In operation S3440, the processor 180 may input, to the Parkinson's disease prediction model, a speaker's speech result corresponding to the audio data for the Parkinson's disease prediction and may obtain a Parkinson's disease prediction result for the speaker as an output of the Parkinson's disease prediction model.

In an alternative embodiment, the processor 180 may generate a gender classification model. In the present embodiment, the gender classification model may be generated by training a deep neural network model pre-trained to classify a speaker's gender by using the audio data including the speaker's speech result. The deep neural network model may be a model that receives the audio data including the speaker's speech result and is trained in a supervised learning method by using training data labeled with the speaker's gender. The processor 180 may verify the accuracy of the gender classification model based on the first different syntactic combinations, the second different syntactic combinations, and the third different syntactic combinations. The processor 180 may determine the third syntactic combination ranked in the highest rank as the audio data for gender classification, based on the result of verifying the accuracy of the gender classification model. The third syntactic combination ranked in the highest rank may be a syntactic combination in which the third different syllables generated by combining the third and eighth consonants according to the consonant order with the certain vowel are repeated a preset number of times. The processor 180 may input, to the gender classification model, a speaker's speech result corresponding to the determined audio data and may obtain a gender classification result for the speaker as an output of the gender classification model.

In an alternative embodiment, the processor 180 may generate a syntactic performance comparison model. In the present embodiment, the syntactic performance comparison model may be generated by training a deep neural network model pre-trained to output a performance comparison result for each syntactic included in the audio data by using the audio data including the speaker's speech result. The deep neural network model may be a model that receives the audio data including the speaker's speech result and is trained in a supervised learning method by using training data labeled with the performance comparison result for each syntactic. The processor 180 may verify the accuracy of the syntactic performance comparison model for the Parkinson's disease prediction, based on certain syntactics extracted from the audio data including the speaker's speech result. The processor 180 may determine a syntactic ranked in a high rank as the audio data for syntactic performance comparison for the Parkinson's disease prediction, based on the result of verifying the accuracy of the syntactic performance comparison model for the Parkinson's disease prediction. The processor 180 may determine the first syntactic ranked in the first rank as the audio data for syntactic performance comparison for the Parkinson's disease prediction, based on the result of verifying the accuracy of the syntactic performance comparison model for the Parkinson's disease prediction. The first syntactic ranked in the first rank may be a syntactic in which the first syllable generated by combining the fourth consonant according to the consonant order with the certain vowel is repeated a preset number of times. The processor 180 may determine the first syntactic ranked in the second rank as the audio data for syntactic performance comparison for the Parkinson's disease prediction. The first syntactic ranked in the second rank may be a syntactic in which the first syllable generated by combining the eighth consonant according to the consonant order with the certain vowel is repeated a preset number of times. The processor 180 may determine the basic vowel set ranked in the third rank as the audio data for syntactic performance comparison for the Parkinson's disease prediction. The processor 180 may determine the first syntactic ranked in the fourth rank as the audio data for syntactic performance comparison for the Parkinson's disease prediction. The first syntactic ranked in the fourth rank may be a syntactic in which the first syllable generated by combining the eleventh consonant according to the consonant order with the certain vowel is repeated a preset number of times. The processor 180 may determine the first syntactic ranked in the fifth rank as the audio data for syntactic performance comparison for the Parkinson's disease prediction. The first syntactic ranked in the fifth rank may be a syntactic in which the first syllable generated by combining the second consonant according to the consonant order with the certain vowel is repeated a preset number of times. The processor 180 may determine the first syntactic ranked in the sixth rank as the audio data for syntactic performance comparison for the Parkinson's disease prediction. The first syntactic ranked in the sixth rank may be a syntactic in which the first syllable generated by combining the sixth consonant according to the consonant order with the certain vowel is repeated a preset number of times. The processor 180 may determine the first syntactic ranked in the seventh rank as the audio data for syntactic performance comparison for the Parkinson's disease prediction. The first syntactic ranked in the seventh rank may be a syntactic in which the first syllable generated by combining the twelfth consonant according to the consonant order with the certain vowel is repeated a preset number of times. The processor 180 may determine the first syntactic ranked in the eighth rank as the audio data for syntactic performance comparison for the Parkinson's disease prediction. The first syntactic ranked in the eighth rank may be a syntactic in which the first syllable generated by combining the fourteenth consonant according to the consonant order with the certain vowel is repeated a preset number of times. The processor 180 may determine the first syntactic ranked in the ninth rank as the audio data for syntactic performance comparison for the Parkinson's disease prediction. The first syntactic ranked in the ninth rank may be a combination of the first syllable generated by combining the thirteenth consonant according to the consonant order with the certain vowel, the first syllable generated by combining the twelfth consonant according to the consonant order with the certain vowel, and the first syllable generated by combining the eleventh consonant according to the consonant order with the certain vowel. The processor 180 may determine the first syntactic ranked in the tenth rank as the audio data for syntactic performance comparison for the Parkinson's disease prediction. The first syntactic ranked in the tenth rank may be a syntactic in which the first syllable generated by combining the fifth consonant according to the consonant order with the certain vowel is repeated a preset number of times. The processor 180 may input, to the syntactic performance comparison model, a speaker's speech result corresponding to the determined audio data and obtain a syntactic performance comparison result for the Parkinson's disease prediction for the speaker as an output of the syntactic performance comparison model.

In an alternative embodiment, the processor 180 may generate a first Parkinson's disease accuracy classification model. In the present embodiment, the first Parkinson's disease accuracy classification model may be generated by training a deep neural network model pre-trained to classify the Parkinson's disease accuracy for the speaker by using the audio data including the speaker's speech result. The deep neural network model may be a model that receives the audio data including the speaker's speech result and is trained in a supervised learning method by using training data labeled with a first class and/or a second class and a third class. In the present embodiment, the first class may include a disease with the highest accuracy from among normal, Parkinson, multiple system atrophy, and cerebellar atrophy included in the Parkinson's disease-related diseases. The second class may include a disease with the second highest accuracy from among normal, Parkinson, multiple system atrophy, and cerebellar atrophy included in the Parkinson's disease-related diseases. The third class may include the remaining Parkinson's disease-related diseases excluding the first class or the second class. The processor 180 may verify the accuracy of the first Parkinson's disease accuracy classification model based on the first different syntactic combinations, the second different syntactic combinations, and the third different syntactic combinations. The processor 180 may determine a syntactic combination ranked in a high rank as the audio data for classifying the Parkinson's disease accuracy, based on the result of verifying the accuracy of the first Parkinson's disease accuracy classification model. The processor 180 may determine the third syntactic combination ranked in the highest rank as the audio data for classifying the Parkinson's disease accuracy, based on the result of verifying the accuracy of the first Parkinson's disease accuracy classification model. The third syntactic combination ranked in the highest rank may correspond to cerebellar atrophy as the first class and the third class including the remaining Parkinson's disease-related diseases excluding cerebellar atrophy. In addition, the third syntactic combination ranked in the highest rank may be a syntactic combination in which the third different syllables generated by combining the third and eighth consonants according to the consonant order with the certain vowel are repeated a preset number of times. The processor 180 may determine the third syntactic combination ranked in the second highest rank as the audio data for classifying the Parkinson's disease accuracy. The third syntactic combination ranked in the second highest rank may correspond to normal as the first class and the third class including the remaining Parkinson's disease-related diseases excluding normal. In addition, the third syntactic combination ranked in the second highest rank may be a syntactic combination in which the third different syllables generated by combining the eighth and fourteenth consonants according to the consonant order with the certain vowel are repeated a preset number of times. The processor 180 may determine the third syntactic combination ranked in the third highest rank as the audio data for classifying the Parkinson's disease accuracy. The third syntactic combination ranked in the third highest rank may correspond to multiple system atrophy as the first class and the third class including the remaining Parkinson's disease-related diseases excluding multiple system atrophy. In addition, the third syntactic combination ranked in the third highest rank may be a syntactic combination in which the third different syllables generated by combining the second and seventh consonants according to the consonant order with the certain vowel are repeated a preset number of times. The processor 180 may determine the third syntactic combination ranked in the fourth highest rank as the audio data for classifying the Parkinson's disease accuracy. The third syntactic combination ranked in the fourth highest rank may correspond to Parkinson as the first class and the third class including the remaining Parkinson's disease-related diseases excluding Parkinson. In addition, the third syntactic combination ranked in the fourth highest rank may be a syntactic combination in which the third different syllables generated by combining the eighth and fourteenth consonants according to the consonant order with the certain vowel are repeated a preset number of times. The processor 180 may input, to the first Parkinson's disease accuracy classification model, a speaker's speech result corresponding to the determined audio data and may obtain a first Parkinson's disease accuracy classification result for the speaker as an output of the first Parkinson's disease accuracy classification model.

In an alternative embodiment, the processor 180 may generate a second Parkinson's disease accuracy classification model. In the present embodiment, the second Parkinson's disease accuracy classification model may be generated by training a deep neural network model pre-trained to classify the Parkinson's disease accuracy for the speaker by using the audio data including the speaker's speech result. The deep neural network model may be a model that receives the audio data including the speaker's speech result and is trained in a supervised learning method by using training data labeled with two or more of a fourth class, a fifth class, and a sixth class. In the present embodiment, the fourth class may include a disease with the highest accuracy from among normal, Parkinson, multiple system atrophy, and cerebellar atrophy included in the Parkinson's disease-related diseases. The fifth class may include a disease with higher accuracy from among the remaining three Parkinson's disease-related diseases excluding the fourth class. The sixth class may include a disease with the highest accuracy from among the remaining two Parkinson's disease-related diseases excluding the fourth class and the fifth class. The processor 180 may verify the accuracy of the second Parkinson's disease accuracy classification model based on the first different syntactic combinations, the second different syntactic combinations, and the third different syntactic combinations. The processor 180 may determine a syntactic combination ranked in a high rank as the audio data for classifying the Parkinson's disease accuracy, based on the result of verifying the accuracy of the second Parkinson's disease accuracy classification model. The processor 180 may determine the first syntactic combination ranked in the first rank as the audio data for classifying the Parkinson's disease accuracy, based on the result of verifying the accuracy of the second Parkinson's disease accuracy classification model. The first syntactic combination ranked in the first rank may correspond to normal as the fourth class and cerebellar atrophy as the fifth class. In addition, the first syntactic combination ranked in the first rank may be a syntactic combination in which the first syllables generated by combining the first to seventh consonants according to the consonant order with the certain vowel are repeated a preset number of times. The processor 180 may determine the first syntactic combination ranked in the second rank as the audio data for classifying the Parkinson's disease accuracy. The first syntactic combination ranked in the second rank may correspond to normal as the fourth class and multiple system atrophy as the fifth class. In addition, the first syntactic combination ranked in the second rank may be a syntactic combination in which the first syllables generated by combining the first to seventh consonants according to the consonant order with the certain vowel are repeated a preset number of times. The processor 180 may determine the first syntactic combination ranked in the third rank as the audio data for classifying the Parkinson's disease accuracy. The first syntactic combination ranked in the third rank may correspond to multiple system atrophy as the fourth class and cerebellar atrophy as the fifth class. In addition, the first syntactic combination ranked in the third rank may be a syntactic combination in which the first syllables generated by combining the first to seventh consonants according to the consonant order with the certain vowel are repeated a preset number of times. The processor 180 may determine the first syntactic combination ranked in the fourth rank as the audio data for classifying the Parkinson's disease accuracy. The first syntactic combination ranked in the fourth rank may correspond to Parkinson as the fourth class and cerebellar atrophy as the fifth class. In addition, the first syntactic combination ranked in the fourth rank may be a syntactic combination in which the first syllables generated by combining the first to seventh consonants according to the consonant order with the certain vowel are repeated a preset number of times. The processor 180 may determine the first syntactic combination ranked in the fifth rank as the audio data for classifying the Parkinson's disease accuracy. The first syntactic combination ranked in the fifth rank may correspond to normal as the fourth class, multiple system atrophy as the fifth class, and cerebellar atrophy as the sixth class. In addition, the first syntactic combination ranked in the fifth rank may be a syntactic combination in which the first syllables generated by combining the first to seventh consonants according to the consonant order with the certain vowel are repeated a preset number of times. The processor 180 may determine the first syntactic combination ranked in the sixth rank as the audio data for classifying the Parkinson's disease accuracy. The first syntactic combination ranked in the sixth rank may correspond to normal as the fourth class and Parkinson as the fifth class. In addition, the first syntactic combination ranked in the sixth rank may be a syntactic combination in which the first syllables generated by combining the first to ninth consonants according to the consonant order with the certain vowel are repeated a preset number of times. The processor 180 may determine the first syntactic combination ranked in the seventh rank as the audio data for classifying the Parkinson's disease accuracy. The first syntactic combination ranked in the seventh rank may correspond to normal as the fourth class, Parkinson as the fifth class, and cerebellar atrophy as the sixth class. In addition, the first syntactic combination ranked in the seventh rank may be a syntactic combination in which the first syllables generated by combining the first to seventh consonants according to the consonant order with the certain vowel are repeated a preset number of times. The processor 180 may determine the first syntactic combination ranked in the eighth rank as the audio data for classifying the Parkinson's disease accuracy. The first syntactic combination ranked in the eighth rank may correspond to Parkinson as the fourth class and multiple system atrophy as the fifth class. In addition, the first syntactic combination ranked in the eighth rank may be a syntactic combination in which the first syllables generated by combining the first to ninth consonants according to the consonant order with the certain vowel are repeated a preset number of times. The processor 180 may determine the first syntactic combination ranked in the ninth rank as the audio data for classifying the Parkinson's disease accuracy. The first syntactic combination ranked in the ninth rank may correspond to normal as the fourth class, Parkinson as the fifth class, and multiple system atrophy as the sixth class. In addition, the first syntactic combination ranked in the ninth rank may be a syntactic combination in which the first syllables generated by combining the first to seventh consonants according to the consonant order with the certain vowel are repeated a preset number of times. The processor 180 may determine the first syntactic combination ranked in the tenth rank as the audio data for classifying the Parkinson's disease accuracy. The first syntactic combination ranked in the tenth rank may correspond to Parkinson as the fourth class, multiple system atrophy as the fifth class, and cerebellar atrophy as the sixth class. In addition, the first syntactic combination ranked in the tenth rank may be a syntactic combination in which the first syllables generated by combining the first to seventh consonants according to the consonant order with the certain vowel are repeated a preset number of times. The processor 180 may input, to the second Parkinson's disease accuracy classification model, a speaker's speech result corresponding to the determined audio data and may obtain a Parkinson's disease accuracy classification result for the speaker as an output of the second Parkinson's disease accuracy classification model.

The embodiments described above may be implemented in the form of a computer program that is executable through various components on a computer, and the computer program may be recorded on a computer-readable recording medium. Examples of the computer-readable recording medium may include a magnetic medium such as hard disk, floppy disk, and magnetic tape, an optical recording medium such as compact disc read-only memory (CD-ROM) and digital versatile disc (DVD), a magnetooptical medium such as floptical disk, and a hardware device particularly configured to store and execute program instructions, such as read-only memory (ROM), random access memory (RAM), and flash memory.

The computer program may be those specially designed and configured for the disclosure or those known to and usable by those of ordinary skill in the field of computer software. Examples of the computer program may include not only machine language code generated by a compiler, but also high-level language code that is executable using an interpreter by a computer.

The use of the term "the" and similar demonstratives in the context of describing the present specification (especially in the context of the claims) is to be construed to cover both the singular and the plural. Furthermore, recitation of ranges of values herein are merely intended to serve as a shorthand method of referring individually to each separate value falling within the range, unless otherwise indicated herein, and each separate value is incorporated into the specification as if it were individually recited herein.

According to the disclosure, the Parkinson's disease prediction apparatus and the Parkinson's disease prediction method, which predict Parkinson's disease for a speaker through audio analysis, may be provided.

The effects of the disclosure are not limited to those described above, and other effects that are not mentioned herein will be clearly understood by those of ordinary skill in the art from the appended claims.

## Claims

1. A Parkinson's disease prediction method, performed by a processor (180) of a Parkinson's disease prediction apparatus (100), the Parkinson's disease prediction method comprising:
extracting syntactic combinations from audio data including a speaker's speech result;
verifying accuracy of a Parkinson's disease prediction model by changing conditions for preprocessing the audio data and the syntactic combinations;
determining a syntactic combination ranked in a high rank as audio data for Parkinson's disease prediction, based on a result of verifying the accuracy of the Parkinson's disease prediction model; and
inputting, to the Parkinson's disease prediction model, a speaker's speech result corresponding to the audio data for the Parkinson's disease prediction and obtaining a Parkinson's disease prediction result for the speaker as an output of the Parkinson's disease prediction model.

2. The Parkinson's disease prediction method of claim 1, wherein the syntactic combinations is a plurality of first different syntactic combinations including at least one first syllable generated by combining a certain consonant with a certain vowel,
the syntactic combinations is a plurality of second different syntactic combinations in which a basic vowel set, a second syllable generated by combining a certain double consonant with a certain vowel, and at least one of the plurality of first different syntactic combinations are combined with each other, or
the syntactic combinations is a plurality of third different syntactic combinations including at least one third syllable generated by combining an arbitrary consonant with an arbitrary vowel and one syntactic combination included in the plurality of second different syntactic combinations.

3. The Parkinson's disease prediction method of claim 2, wherein the certain consonant included in the plurality of first different syntactic combinations is combined with the certain vowel in a consonant order (201) according to a language regulation, and
the certain vowel included in the plurality of first different syntactic combinations is determined as a single vowel.

4. The Parkinson's disease prediction method of claim 3, wherein the extracting of the syntactic combinations from the audio data comprises extracting, from the audio data, the plurality of first different syntactic combinations in which the at least one first syllable generated by combining n^{th} to (n+k)^{th} consonants according to the consonant order (201) with the certain vowel is repeated a preset number of times, wherein n includes a natural number and k includes 0 and a natural number.

5. The Parkinson's disease prediction method of claim 4, further comprising, before the extracting of the syntactic combinations, generating the Parkinson's disease prediction model,
wherein the Parkinson's disease prediction model is generated by training a deep neural network model pre-trained to predict Parkinson's disease for the speaker by using the audio data including the speaker's speech result, and
the deep neural network model is a model that receives the audio data including the speaker's speech result and is trained in a supervised learning method by using training data labeled with one of normal, Parkinson, multiple system atrophy, and cerebellar atrophy included in Parkinson's disease-related diseases.

6. The Parkinson's disease prediction method of claim 5, wherein the verifying of the accuracy comprises verifying the accuracy of the Parkinson's disease prediction model based on a second preprocessing condition for preprocessing the audio data and the plurality of second different syntactic combinations, and
the second preprocessing condition comprises a condition for executing first acoustic preprocessing to unify channels for recording the audio data and unify sample rates of the audio data.

7. The Parkinson's disease prediction method of claim 6, wherein the determining as the audio data comprises determining the basic vowel set ranked in a highest rank as the audio data for the Parkinson's disease prediction, based on the result of verifying the accuracy of the Parkinson's disease prediction model.

8. The Parkinson's disease prediction method of claim 5, wherein the verifying of the accuracy comprises verifying the accuracy of the Parkinson's disease prediction model based on a seventh preprocessing condition for preprocessing the audio data, the plurality of first different syntactic combinations, and the plurality of second different syntactic combinations, and
the seventh preprocessing condition comprises a condition for executing second acoustic preprocessing to unify channels for recording the audio data, unify sample rates of the audio data, normalize the audio data according to an average volume, filter the audio data in a preset band, remove a DC-offset from the audio data, and reduce noise from the audio data, and use of edge from among types of data padding that equalizes a size of the audio data by filling a silent section of the audio data with a specific value.

9. The Parkinson's disease prediction method of claim 8, wherein the determining as the audio data comprises determining a first syntactic combination ranked in a highest rank and a first syntactic combination ranked in a second highest rank as the audio data for the Parkinson's disease prediction, based on a result of verifying the accuracy of the Parkinson's disease prediction model,
the first syntactic combination ranked in the highest rank is a syntactic combination in which the at least one first syllable generated by combining first to ninth consonants according to the consonant order (201) with the certain vowel is repeated a predetermined number of times,
the first syntactic combination ranked in the second highest rank is a syntactic combination in which the at least one first syllable generated by combining first to seventh consonants according to the consonant order (201) with the certain vowel is repeated a predetermined number of times.

10. A computer-readable recording medium having recorded thereon a computer program comprising instructions which, when executed by a computer, cause the computer to perform the method of claim 1.

11. A Parkinson's disease prediction apparatus (100) comprising:
a processor (180); and
a memory (190) operatively connected to the processor (180) and configured to store at least one code executable by the processor (180),
wherein, when executed by the process, the at least one code causes the processor (180) to:
extract syntactic combinations from audio data including a speaker's speech result;
verify accuracy of a Parkinson's disease prediction model by changing conditions for preprocessing the audio data and the syntactic combinations;
determine a syntactic combination ranked in a high rank as audio data for Parkinson's disease prediction, based on a result of verifying the accuracy of the Parkinson's disease prediction model; and
input, to the Parkinson's disease prediction model, a speaker's speech result corresponding to the audio data for the Parkinson's disease prediction and obtain a Parkinson's disease prediction result for the speaker as an output of the Parkinson's disease prediction model.

## Patentansprüche

1. Verfahren zur Vorhersage der Parkinson-Krankheit, das von einem Prozessor (180) einer Vorrichtung (100) zur Vorhersage der Parkinson-Krankheit durchgeführt wird, wobei das Verfahren zur Vorhersage der Parkinson-Krankheit Folgendes umfasst:
Extrahieren syntaktischer Kombinationen aus Audiodaten, die ein Sprechergebnis eines Sprechers enthalten;
Überprüfen einer Genauigkeit eines Modells zur Vorhersage der Parkinson-Krankheit durch Ändern von Bedingungen zum Vorverarbeiten der Audiodaten und der syntaktischen Kombinationen;
Bestimmen einer als hochrangig eingestuften syntaktischen Kombination als Audiodaten für eine Vorhersage der Parkinson-Krankheit auf Grundlage eines Ergebnisses des Überprüfens der Genauigkeit des Modells zur Vorhersage der Parkinson-Krankheit; und
Eingeben eines Sprechergebnisses eines Sprechers, das den Audiodaten für die Vorhersage der Parkinson-Krankheit entspricht, in das Modell zur Vorhersage der Parkinson-Krankheit und Erhalten eines Vorhersageergebnisses für die Parkinson-Krankheit für den Sprecher als Ausgabe des Modells zur Vorhersage der Parkinson-Krankheit.

2. Verfahren zur Vorhersage der Parkinson-Krankheit nach Anspruch 1, wobei es sich bei den syntaktischen Kombinationen um eine Vielzahl erster unterschiedlicher syntaktischer Kombinationen handelt, die mindestens eine erste Silbe enthalten, die durch Kombinieren eines bestimmten Konsonanten mit einem bestimmten Vokal erzeugt wird,
es sich bei den syntaktischen Kombinationen um eine Vielzahl zweiter unterschiedlicher syntaktischer Kombinationen, in denen ein grundlegender Satz von Vokalen, eine zweite Silbe, die durch Kombinieren eines bestimmten Doppelkonsonanten mit einem bestimmten Vokal erzeugt wird, und mindestens eine der Vielzahl erster unterschiedlicher syntaktischer Kombinationen miteinander kombiniert werden, handelt oder
es sich bei den syntaktischen Kombinationen um eine Vielzahl dritter unterschiedlicher syntaktischer Kombinationen handelt, die mindestens eine dritte Silbe, die durch Kombinieren eines beliebigen Konsonanten mit einem beliebigen Vokal erzeugt wird, und eine syntaktische Kombination enthalten, die in der Vielzahl zweiter unterschiedlicher syntaktischer Kombinationen enthalten ist.

3. Verfahren zur Vorhersage der Parkinson-Krankheit nach Anspruch 2, wobei der bestimmte Konsonant, der in der Vielzahl erster unterschiedlicher syntaktischer Kombinationen enthalten ist, mit dem bestimmten Vokal in einer Konsonantenreihenfolge (201) gemäß einer Sprachregelung kombiniert wird und
der bestimmte Vokal, der in der Vielzahl erster unterschiedlicher syntaktischer Kombinationen enthalten ist, als einzelner Vokal bestimmt wird.

4. Verfahren zur Vorhersage der Parkinson-Krankheit nach Anspruch 3, wobei das Extrahieren der syntaktischen Kombinationen aus den Audiodaten Extrahieren, aus den Audiodaten, der Vielzahl erster unterschiedlicher syntaktischer Kombinationen umfasst, in denen die mindestens eine erste Silbe, die durch Kombinieren n-ter bis (n+k)-ter Konsonanten gemäß der Konsonantenreihenfolge (201) mit dem bestimmten Vokal erzeugt wird, eine voreingestellte Anzahl von Malen wiederholt wird, wobei n eine natürliche Zahl und k 0 und eine natürliche Zahl umfasst.

5. Verfahren zur Vorhersage der Parkinson-Krankheit nach Anspruch 4, ferner umfassend vor dem Extrahieren der syntaktischen Kombinationen Erzeugen des Modells zur Vorhersage der Parkinson-Krankheit, wobei das Modell zur Vorhersage der Parkinson-Krankheit durch Trainieren eines DNN- (deep neural network, tiefes neuronales Netz) Modells erzeugt wird, das vorab trainiert wurde, um die Parkinson-Krankheit für den Sprecher unter Verwendung der Audiodaten einschließlich des Sprechergebnisses des Sprechers vorherzusagen, und das DNN-Modell ein Modell ist, das die Audiodaten einschließlich des Sprechergebnisses des Sprechers empfängt und in einem überwachten Lernverfahren unter Verwendung von Trainingsdaten trainiert wird, die mit einem von "normal", "Parkinson", "multiple Systematrophie" und "Kleinhirnatrophie", die zu mit Parkinson in Verbindung gebrachten Erkrankungen gehören, gekennzeichnet sind.

6. Verfahren zur Vorhersage der Parkinson-Krankheit nach Anspruch 5, wobei das Überprüfen der Genauigkeit Überprüfen der Genauigkeit des Modells zur Vorhersage der Parkinson-Krankheit auf Grundlage einer zweiten Vorverarbeitungsbedingung zum Vorverarbeiten der Audiodaten und der Vielzahl zweiter unterschiedlicher syntaktischer Kombinationen umfasst und
die zweite Vorverarbeitungsbedingung eine Bedingung zum Ausführen einer ersten akustischen Vorverarbeitung zum Vereinheitlichen von Kanälen zum Aufzeichnen der Audiodaten und zum Vereinheitlichen von Abtastraten der Audiodaten umfasst.

7. Verfahren zur Vorhersage der Parkinson-Krankheit nach Anspruch 6, wobei das Bestimmen als Audiodaten Bestimmen des als höchstrangig eingestuften grundlegenden Vokalsatzes als Audiodaten für die Vorhersage der Parkinson-Krankheit auf Grundlage des Ergebnisses des Überprüfens der Genauigkeit des Modells zur Vorhersage der Parkinson-Krankheit umfasst.

8. Verfahren zur Vorhersage der Parkinson-Krankheit nach Anspruch 5, wobei das Überprüfen der Genauigkeit Überprüfen der Genauigkeit des Modells zur Vorhersage der Parkinson-Krankheit auf Grundlage einer siebten Vorverarbeitungsbedingung zum Vorverarbeiten der Audiodaten, der Vielzahl erster unterschiedlicher syntaktischer Kombinationen und der Vielzahl zweiter unterschiedlicher syntaktischer Kombinationen umfasst und
die siebte Vorverarbeitungsbedingung eine Bedingung zum Ausführen einer zweiten akustischen Vorverarbeitung umfasst, um Kanäle zum Aufzeichnen der Audiodaten zu vereinheitlichen, Abtastraten der Audiodaten zu vereinheitlichen, die Audiodaten gemäß einer durchschnittlichen Lautstärke zu normalisieren, die Audiodaten in einem voreingestellten Band zu filtern, einen Gleichstromversatz aus den Audiodaten zu entfernen und Rauschen aus den Audiodaten zu reduzieren, und die Verwendung eines Randes unter Arten von Datenauffüllung, die eine Größe der Audiodaten durch Füllen eines stillen Abschnitts der Audiodaten mit einem bestimmten Wert ausgleicht.

9. Verfahren zur Vorhersage der Parkinson-Krankheit nach Anspruch 8, wobei das Bestimmen als Audiodaten Bestimmen einer als höchstrangig eingestuften ersten syntaktischen Kombination und einer als zweithöchstrangig eingestuften ersten syntaktischen Kombination als Audiodaten zur Vorhersage der Parkinson-Krankheit auf Grundlage eines Ergebnisses des Überprüfens der Genauigkeit des Modells zur Vorhersage der Parkinson-Krankheit umfasst,
die als höchstrangig eingestufte erste syntaktische Kombination eine syntaktische Kombination ist, in der die mindestens eine erste Silbe, die durch Kombinieren eines ersten bis neunten Konsonanten gemäß der Konsonantenreihenfolge (201) mit dem bestimmten Vokal erzeugt wird, eine vorbestimmte Anzahl von Malen wiederholt wird,
die als zweithöchstrangig eingestufte erste syntaktische Kombination eine syntaktische Kombination ist, in der die mindestens eine erste Silbe, die durch Kombinieren eines ersten bis siebten Konsonanten gemäß der Konsonantenreihenfolge (201) mit dem bestimmten Vokal erzeugt wird, eine vorbestimmte Anzahl von Malen wiederholt wird.

10. Computerlesbares Aufzeichnungsmedium, auf dem ein Computerprogramm aufgezeichnet ist, das Befehle umfasst, die bei Ausführung durch einen Computer bewirken, dass der Computer das Verfahren nach Anspruch 1 durchführt.

11. Vorrichtung (100) zur Vorhersage der Parkinson-Krankheit, umfassend:
einen Prozessor (180); und
einen Speicher (190), der betriebsmäßig mit dem Prozessor (180) verbunden und so konfiguriert ist, dass er mindestens einen durch den Prozessor (180) ausführbaren Code speichert,
wobei der mindestens eine Code bei Ausführung durch den Prozessor bewirkt, dass der Prozessor (180):
syntaktische Kombinationen aus Audiodaten extrahiert, die ein Sprechergebnis eines Sprechers enthalten;
eine Genauigkeit eines Modells zur Vorhersage der Parkinson-Krankheit durch Ändern von Bedingungen zum Vorverarbeiten der Audiodaten und der syntaktischen Kombinationen überprüft;
eine als hochrangig eingestufte syntaktische Kombination als Audiodaten zur Vorhersage der Parkinson-Krankheit auf Grundlage eines Ergebnisses des Überprüfens der Genauigkeit des Modells zur Vorhersage der Parkinson-Krankheit bestimmt; und
ein Sprechergebnis eines Sprechers, das den Audiodaten zur Vorhersage der Parkinson-Krankheit entspricht, in das Modell zur Vorhersage der Parkinson-Krankheit eingibt und ein Vorhersageergebnis der Parkinson-Krankheit für den Sprecher als Ausgabe des Modells zur Vorhersage der Parkinson-Krankheit erhält.

## Revendications

1. Procédé de prédiction de la maladie de Parkinson, effectué par un processeur (180) d'un appareil de prédiction de la maladie de Parkinson (100), le procédé de prédiction de la maladie de Parkinson comprenant :
l'extraction de combinaisons syntaxiques à partir de données audio incluant un résultat de paroles d'un locuteur ;
la vérification de la précision d'un modèle de prédiction de la maladie de Parkinson en changeant des conditions de prétraitement des données audio et des combinaisons syntaxiques ;
la détermination d'une combinaison syntaxique classée à un rang élevé en tant que données audio pour la prédiction de la maladie de Parkinson, sur la base d'un résultat de vérification de la précision du modèle de prédiction de la maladie de Parkinson ; et
l'entrée, dans le modèle de prédiction de la maladie de Parkinson, d'un résultat de paroles d'un locuteur correspondant aux données audio pour la prédiction de la maladie de Parkinson et l'obtention d'un résultat de prédiction de la maladie de Parkinson pour le locuteur en tant que sortie du modèle de prédiction de la maladie de Parkinson.

2. Procédé de prédiction de la maladie de Parkinson selon la revendication 1, dans lequel les combinaisons syntaxiques sont une pluralité de premières combinaisons syntaxiques différentes incluant au moins une première syllabe générée en combinant une certaine consonne avec une certaine voyelle,
les combinaisons syntaxiques sont une pluralité de deuxièmes combinaisons syntaxiques différentes dans lesquelles un ensemble de voyelles de base, une deuxième syllabe générée en combinant une certaine double consonne avec une certaine voyelle, et au moins une de la pluralité de premières combinaisons syntaxiques différentes sont combinés entre eux, ou
les combinaisons syntaxiques sont une pluralité de troisièmes combinaisons syntaxiques différentes incluant au moins une troisième syllabe générée en combinant une consonne arbitraire avec une voyelle arbitraire et une combinaison syntaxique incluse dans la pluralité de deuxièmes combinaisons syntaxiques différentes.

3. Procédé de prédiction de la maladie de Parkinson selon la revendication 2, dans lequel la certaine consonne incluse dans la pluralité de premières combinaisons syntaxiques différentes est combinée avec la certaine voyelle dans un ordre consonantique (201) selon une réglementation linguistique, et
la certaine voyelle incluse dans la pluralité de premières combinaisons syntaxiques différentes est déterminée comme une voyelle unique.

4. Procédé de prédiction de la maladie de Parkinson selon la revendication 3, dans lequel l'extraction des combinaisons syntaxiques à partir des données audio comprend l'extraction, à partir des données audio, de la pluralité de premières combinaisons syntaxiques différentes dans lesquelles l'au moins une première syllabe générée en combinant les nième à (n+k)^{ième} consonnes selon l'ordre consonantique (201) avec la certaine voyelle est répétée un nombre prédéfini de fois, dans lequel n inclut un nombre naturel et k inclut 0 et un nombre naturel.

5. Procédé de prédiction de la maladie de Parkinson selon la revendication 4, comprenant en outre, avant l'extraction des combinaisons syntaxiques, la génération du modèle de prédiction de la maladie de Parkinson,
dans lequel le modèle de prédiction de la maladie de Parkinson est généré en entraînant un modèle de réseau neuronal profond préentraîné pour prédire la maladie de Parkinson pour le locuteur en utilisant les données audio incluant le résultat de paroles du locuteur, et
le modèle de réseau neuronal profond est un modèle qui reçoit les données audio incluant le résultat de paroles du locuteur et est entraîné selon une méthode d'apprentissage supervisé en utilisant des données d'entraînement étiquetées avec l'un des éléments suivants : normal, Parkinson, atrophie multisystématisée, et atrophie cérébelleuse incluse dans des maladies liées à la maladie de Parkinson.

6. Procédé de prédiction de la maladie de Parkinson selon la revendication 5, dans lequel la vérification de la précision comprend la vérification de la précision du modèle de prédiction de la maladie de Parkinson sur la base d'une deuxième condition de prétraitement pour prétraiter les données audio et la pluralité de deuxièmes combinaisons syntaxiques différentes, et
la deuxième condition de prétraitement comprend une condition pour exécuter un premier prétraitement acoustique afin d'unifier des canaux pour enregistrer les données audio et d'unifier des taux d'échantillonnage des données audio.

7. Procédé de prédiction de la maladie de Parkinson selon la revendication 6, dans lequel la détermination en tant que données audio comprend la détermination de l'ensemble de voyelles de base classé à un rang le plus élevé en tant que données audio pour la prédiction de la maladie de Parkinson, sur la base du résultat de la vérification de la précision du modèle de prédiction de la maladie de Parkinson.

8. Procédé de prédiction de la maladie de Parkinson selon la revendication 5, dans lequel la vérification de la précision comprend la vérification de la précision du modèle de prédiction de la maladie de Parkinson sur la base d'une septième condition de prétraitement pour prétraiter les données audio, la pluralité de premières combinaisons syntaxiques différentes et la pluralité de deuxièmes combinaisons syntaxiques différentes, et
la septième condition de prétraitement comprend une condition pour exécuter un deuxième prétraitement acoustique afin d'unifier des canaux pour enregistrer les données audio, d'unifier des taux d'échantillonnage des données audio, de normaliser les données audio selon un volume moyen, de filtrer les données audio dans une bande prédéfinie, de supprimer un décalage en continu des données audio et de réduire le bruit des données audio, et d'utiliser un bord parmi des types de remplissage de données qui égalise une taille des données audio en remplissant une section silencieuse des données audio avec une valeur spécifique.

9. Procédé de prédiction de la maladie de Parkinson selon la revendication 8, dans lequel la détermination en tant que données audio comprend la détermination d'une première combinaison syntaxique classée à un rang le plus élevé et d'une première combinaison syntaxique classée à un deuxième rang le plus élevé en tant que données audio pour la prédiction de la maladie de Parkinson, sur la base d'un résultat de vérification de la précision du modèle de prédiction de la maladie de Parkinson,
la première combinaison syntaxique classée au rang le plus élevé est une combinaison syntaxique dans laquelle l'au moins une première syllabe générée en combinant des première à neuvième consonnes selon l'ordre consonantique (201) avec la certaine voyelle est répétée un nombre prédéterminé de fois,
la première combinaison syntaxique classée au deuxième rang le plus élevé est une combinaison syntaxique dans laquelle l'au moins une première syllabe générée en combinant des première à septième consonnes selon l'ordre consonantique (201) avec la certaine voyelle est répétée un nombre prédéterminé de fois.

10. Support d'enregistrement lisible par ordinateur sur lequel est enregistré un programme d'ordinateur comprenant des instructions qui, lorsqu'elles sont exécutées par un ordinateur, amènent l'ordinateur à effectuer le procédé de la revendication 1.

11. Appareil (100) de prédiction de la maladie de Parkinson comprenant :
un processeur (180) ; et
une mémoire (190) connectée de manière opérationnelle au processeur (180) et configurée pour stocker au moins un code exécutable par le processeur (180),
dans lequel, lorsqu'il est exécuté par le processus, l'au moins un code amène le processeur (180) à :
extraire des combinaisons syntaxiques à partir de données audio incluant un résultat de paroles d'un locuteur ;
vérifier la précision d'un modèle de prédiction de la maladie de Parkinson en changeant des conditions de prétraitement des données audio et des combinaisons syntaxiques ;
déterminer une combinaison syntaxique classée à un rang élevé en tant que données audio pour la prédiction de la maladie de Parkinson, sur la base d'un résultat de vérification de la précision du modèle de prédiction de la maladie de Parkinson ; et
entrer, dans le modèle de prédiction de la maladie de Parkinson, un résultat de paroles d'un locuteur correspondant aux données audio pour la prédiction de la maladie de Parkinson et obtenir un résultat de prédiction de la maladie de Parkinson pour le locuteur en tant que sortie du modèle de prédiction de la maladie de Parkinson.
